Europäisches Patentamt

(19) European Patent Office

Office européen des brevets

(11) **EP 0 965 596 A1**

(12) **EUROPEAN PATENT APPLICATION**
published in accordance with Art. 158(3) EPC

(43) Date of publication:
22.12.1999 Bulletin 1999/51

(21) Application number: 97949250.1

(22) Date of filing: 26.12.1997

(51) Int. Cl.$^6$: **C07K 14/65**, A61K 45/00,
A61K 38/30, A61K 39/395
// A61K31/35, A61K31/70,
A61K31/725

(86) International application number:
PCT/JP97/04881

(87) International publication number:
WO 98/29451 (09.07.1998 Gazette 1998/27)

(84) Designated Contracting States:
AT BE CH DE DK ES FI FR GB GR IE IT LI LU MC
NL PT SE

(30) Priority: 27.12.1996 JP 34996896

(71) Applicant:
DAIICHI PHARMACEUTICAL CO., LTD.
Chuo-ku, Tokyo 103 (JP)

(72) Inventors:
• SAKANO, Katsuichi,
Daiichi Pharmaceutical Co., Ltd
Tokyo 134 (JP)
• HIGASHIHASHI, Nobuyuki,
Daiichi Pharm. Co.,Ltd
Tokyo 134 (JP)
• HASHIMOTO, Ryuji,
Daiichi Pharmaceutical Co., Ltd
Edogawa-ku, Tokyo 134 (JP)

(74) Representative:
Grünecker, Kinkeldey,
Stockmair & Schwanhäusser
Anwaltssozietät
Maximilianstrasse 58
80538 München (DE)

(54) **METHOD FOR ELEVATING THE CONCENTRATION OF FREE INSULIN-LIKE GROWTH FACTOR**

(57)     A method for elevating the concentration of a free insulin-like growth factor (IGF) by converting IGF contained in living bodies into free IGF having a sufficient IGF activity; a method for elevating the concentration of the IGF/IGFP (insulin-like growth factor-binding protein) complex in living bodies; a substance enabling the elevation of the above concentrations; drugs containing this substance; and a method for screening this substance. Thus, the IGF effect is expressed by administering not IGF externally but the substance capable of increasing free IGF in living bodies. This substance is useful as a preventive and/or remedy for diseases which can be prevented and/or treated by using IGF, such as diabetes, amyotrophic lateral sclerosis and osteoporosis.

EP 0 965 596 A1

## Description

Technical Field

The present invention relates to a method for effectively using endogenous insulin-like growth factors existing much in living bodies.

Background Art

Insulin-like growth factors (which will hereinafter be abbreviated as "IGF") have two molecular types and they are called IGF-I and IGF-II, respectively. Human IGF-I and IGF-II are formed of 70 and 67 amino acids, respectively. Compared with IGF-II, IGF-I has three more amino acids at the site corresponding to C peptide which is a partial structure of insulin. The homology of the amino acid sequence between IGF-I and IGF-II is about 60%, while that between IGF-I and insulin is about 40%. Although the main production site of IGF-I in living bodies was presumed to be the lever/kidney, the analytical results by the northern blotting method using mRNA revealed that IGF-I is produced in almost all the sites in the body tissues (D'Ercole, A. J., et al., Proc. Natl. Acad. Sci. USA., 81, 935 (1984); Humbel, R. E., et al., Eur. J. Biochem., 190, 445 (1990)). Namely, IGF-I acts not only as endocrine but also as paracrine or autocrine.

For IGF-I and IGF-II, there is respective specific receptors, that is, an IGF-I receptor and an IGF-II/cation-independent mannose-6-phosphate receptor. However, IGF-II also binds to the IGF-I receptor and therefore it is presumed that various biological activities of IGF-II are phenomena which appear mainly through the IGF-I receptor on a cellular surface (Casella, S. J., et al., J. Biol. Chem., 261, 9268 (1986); Sakano, K., et al., J. Biol. Chem., 266, 20626 (1991)).

Judging from the amino acid sequence, the IGF-I receptor has a high homology with the insulin receptor and they resemble each other in the intracellular signal transduction mechanism (Shemer, J., et al., J. Biol. Chem., 262, 15476 (1987); Myers, M. G. Jr., et al., Endocrinology, 132, 1421 (1993)). IGFs regulate glucose metabolism predominantly in the peripheral tissue, which is different from insulin, as shown by animal experiments. It thus seems that the receptors of IGFs and insulin are differentially localized in the tissue, which may distinguish the biological effect of IGFs and insulin in the body (Laager, R., et al., J. Clin. Invest., 92, 1903 (1993)).

It is said that the blood of a human adult contains about 100 nM of IGF and about 100 pM of insulin on average (Baxter, R. C., in Modern Concepts of Insulin-Like Growth Factors (Spencer, E. M., ed) pp.371, Elsevier Science Publishing Co., New York-Amsterdam (1991)). Most of IGFs existing in living bodies form complexes with an IGF-binding protein (which will hereinafter be abbreviated as "IGFBP") which is a specific binding protein to each of them. The hypoglycemic activity of free IGF which does not bind to IGFBP is about 5 to 10% of that of insulin (Guler, H. P. et al., New Engl. J. Med., 317, 137 (1987)), indicating that judging only from the hypoglycemic activity, insulin-like growth factors exist in living bodies in an amount of about 50 to 100-folds as much as insulin (Baxter, R. C., in Modern Concepts of Insulin-Like Growth Factors (Spencer, E. M., ed) pp.371, Elsevier Science Publishing Co., New York-Amsterdam (1991)).

Diabetes mellitus is now divided, in accordance with the classification of its clinical image by WHO, roughly into the following three groups:

(1) Insulin-dependent diabetes mellitus (which will hereinafter be abbreviated as "IDDM")
(2) Non insulin dependent diabetes mellitus (which will hereinafter be abbreviated as "NIDDM")
(3) Other diabetes mellitus (derived from pancreato-pathy diseases or endocrinopathy)

A main treating method for IDDM is insulin therapy, while diet therapy, kinesitherapy, or treatment with an oral hypoglycemic agent or with insulin is mainly adopted for NIDDM. In recent years, IGF-I therapy have been under investigation in insulin-dependent diabetes mellitus for which administration of insulin can not bring about effects (Kuzuya, H., et al., Diabetes, 42, 696 (1993)). Also in NIDDM, effects of the treatment with IGF have been under investigation (Zenobi, P. D., et al., J. Clin. Invest., 90, 2234 (1992); Moses, A. C., et al., Diabetes, 45, 91(1996)).

Guler et al. observed that the intravenous injection of IGF-I to human adult in an amount of 100 µg/kg lowered the blood glucose level with the passage of time and after 20 minutes, the blood glucose reached the lowest level (Guler, H. P., et al., New Engl. J. Med., 317, 137 (1987)).

Takano et al. observed that hypoglycemic activity appeared by subcutaneous injection of IGF-I in an amount of 60 to 120 µg/kg to human adult; and that administration of IGF-I for every 6 days in an amount of 100 µg/kg lowered the uric acid level and creatinine level in blood (Takano, K., et al., Endocrinol. Jpn., 37, 309 (1990)).

In addition, there are reports on the lowering in the free fatty acid level in blood (Turkalj. I., et al., J. Clin. Endocrinol. Metab., 75, 1186 (1992)), lowering in the level of neutral fats such as triglyceride (Turkalj. I., et al., J. Clin. Endocrinol. Metab., 75, 1186 (1992); Zenobi, P. D., et al., J. Clin. Invest., 90, 2234 (1992)), lowering in the total cholesterol level (Zenobi, P. D., et al., Diabetologia, 36, 465 (1993)) and an increase of the renal blood flow and a glomerular filtration rate (Elahi, D., et al., in Modern Concepts of Insulin-Like Growth Factors (Spencer, E. M., ed) pp219, Elsevier Science Publishing Co., New York-Amsterdam (1991)), each brought about by the administration of IGF-I.

There is also a report that the administration of IGF-II was effective for intractable diabetes mellitus (Usara, A., et al., Diabetes, 44, Suppl. 1, 33A, 1995)). Moreover, the experiment using model animals suggests the effectiveness of the administration of IGF-I for the purpose of relaxing the stress conditions including glucose metabolism at the time of hemorrhagic shock, alleviating side effects caused by the use of sugar infusion and supplying energy (Unexamined Japanese Patent Publication (KOKAI) No. Hei 7-242565).

Various actions of IGF including not only hypoglycemic action but also cell proliferating action, cell differentiating action, anobolic action are investigated by administering IGF to animals or human beings. It has been found that local administration of IGF-I to the injured peripheral nervous system proliferates the related non-neural cell while stimulating neurons. It is reported that IGF-I receptors are recognized to exist in the spinal cells and administration of IGF-I decreases the cell death of motor neuron. In addition, it is recognized that the administration of IGF increases the muscular end plate, promotes the functional recovery when the sciatic nerve is damaged or prevents peripheral motor paralysis observed at the chemotherapy (Sjoberg, J., et al., Brain Res., 485, 102 (1989).

Based on these experimental results, clinical tests of IGF-I for amyotrophic lateral sclerosis and degenerative diseases of motor neuron have been carried out (Lewis, M. E., et al., Exp. Neurol., 124, 73(1993)). Similarly, the use of the action of IGF has been considered for promoting the survival of neuron cells which is recognized in Alzheimer's disease, apoplexy, amyotrophic lateral sclerosis, Parkinson disease and the like (Unexamined Japanese Patent Publication (KOHYO) No. Hei 6-510305). In addition, effectiveness of IGF-I for muscular dystrophy is also reported (Vlachopapadopoulou, E., et al., J. Clin. Endocrinol. Metab., 80, 3715 (1995)).

The action of IGF to diabetic neuropathy has been studied. It is reported that in an STZ-rat (streptozotocin-diabetic rat) which is an IDDM model rat, alleviation of diabetic neuropathy is observed when IGF is administered in an amount within an extent not causing lowering of the blood glucose level (Zhuang, H-X, et al., Exp. Neurol., 140, pp198-205 (1996)). It is also reported that in the sciatic nerves, spinal nerves and brain nerves of a diabetic obese Zuker (fa/fa) rat which is an NIDDM model rat, the lowering of the IGF-II mRNA level is observed and in addition, alleviation in the diabetic neuropathy is observed when IGF-II is administered in an amount within an extent not causing a decrease in blood glucose level (Zhuang, H-X, et al., J. Pharmacol. Exp. Ther., 283, pp366-374 (1997)). These findings suggest the therapy with IGF is effective for the treatment of diabetic neuropathy.

With regards to cardiac function, it has been studied whether the IGF-I administration alleviates myocardiopathy or not. Described specifically, it is reported that when doxorubicin was administered to a rat to cause it myocardiopathy and its cardiac output was studied, the administration of IGF-I improve the injured myocardial function (Ambler, G. R., et al., Cardiovasc. Res., 27, 1368 (1993)). Similarly, IGF-I has been used with a view to preventing or treating myocardiopathy including myocarditis and myocardial infarction, cardiac disease or acute attack; or increasing cardiac pulsebeats, thereby improving a cardiac output (Unexamined Japanese Patent Publication (KOHYO) No. Hei 6-504286).

There is a report on the investigation of the effects of IGF-I on an experimental animal caused to suffer from acute renal insufficiency by ischemia. From day 5 after ischemia, IGF-I was administered three times a day by subcutaneous injection for three days. As a result, it is reported that compared with the control, the administration brought about an improvement in the renal function, promoted formation of new renal tublar, inhibited proteolysis and promoted protein synthesis and as a whole, decreased catabolism (Ding, H., et al., J. Clin. Invest., 91, 2281 (1993)).

It is also reported that the local administration of IGF-I to the injured skin (wound, burn injury or the like) reduces the term necessary for recovery. According to the report, as a result of the experiment using a rat suffering from burn injury, the administration of rat IGF-I increased its body weight, weight of the enteromucosa, mucosa DNA and protein amount and decreased the transfer of enterobacterium to the intestinal lymph gland, thereby improving the intestinal function and as a whole, brought about good results in life prognosis (Huang, K. F., et al., Arch. Surg., 128, 47 (1993)).

Together with a platelet-derived growth factor (which will hereinafter be abbreviated as "PDGF"), IGF-I promotes the mitosis and protein synthesis of cultured mesenchymal cells; and although curing of skin disorders is not promoted by the single use of PDGF or IGF-I, use of both factors in combination promotes the growth of the connective tissue and epithelial tissue (Stiles, C. D., et al., Proc. Natl. Acad. Sci. USA, 76, 1279 (1987)). Another report, however, says that the single application of one of these growth factors stimulates the wound healing (Tsuboi, R., et al., J. Exp. Med., 172, 245 (1990)). Therefore, there have been attempts to use IGF for the purpose of promoting wound healing (Unexamined Japanese Patent Publication (KOKAI) No. Sho 63-233925, Unexamined Japanese Patent Publication (KOHYO) Nos. Hei 3-505870 and Hei 6-506191, and Unexamined Japanese Patent Publication (KOKAI) No. Hei 7-316066).

In addition, IGF-I is effective for improving immune capacity. IGF-I locally produced in the tissue of thymus or inflammatory site is considered to take part in the proliferation and functional improvement of T lymphocytes having an IGF-I receptor (Tapson, V. F., et al., J. Clin. Invest., 82, 950 (1988)). It is reported that IGF-I is a proliferation promoting factor of lymphocytes at the concentration range of nano molar (Schimpff, R. M., et al., Acta Endocrinol. 102, 21 (1983)). Accordingly, the use of IGF-I for immunodeficient patients including AIDS patients is under investigation (Unexamined Japanese Patent Publication (KOHYO) No. Hei 6-508830).

Moreover, IGF-I is considered to be effective for the treatment of osteoporosis because it has bone mass increasing activity (Bennett, A. E., et al., J. Clin. Endocrinol. Metab., 59, 701 (1984); Brixen, K., et al., J. Bone. Miner. Res., 5, 609 (1990); Johannsson, A. G., et al., J. Intern. Med., 234, 553 (1993); Johannsson, A. G., et al., Lancet, 339, 1619 (1992); Riggs, B. L., Am. J. Med., 95, Suppl.5A, 62S, (1993); Unexamined Japanese Patent Publication (KOKAI) No. Hei 4-235135 and U.S. Patent No. 4,861,757).

It is however known that in nature, almost all the IGFs form their complexes with IGFBP in living bodies, thereby regulating their physiological action (Rechler, M. M., Vitam. Harm., 47, 1 (1993); Clemmons, D. R., Growth Regul., 2, 80 (1992)).

It has so far been confirmed that there exist 6 kinds of IGFBPs and they have been designated "IGFBP-1 to IGFBP-6", respectively. These six kinds of IGFBPs have high homology each other in their amino acid sequence. The homology is marked in the domain at the N-terminal side and C-terminal side containing much cysteine, while homology is not recognized so much in the intermediate part on the amino acid sequence of IGFBP. In the case of human beings, the positions of 16 cysteines are maintained in these six kinds of IGFBPs (in the five kinds of IGFBPs from IGFBP-1 to IGFBP-5, 18 cysteines are positionally maintained) (Shimasaki, S., et al., Prog. Growth Factor Res., 3, 243 (1991)).

The concentrations of IGFBP-1, IGFBP-2 and IGFBP-3 in the blood of the human adult is about 2 nM, 5 nM and 100 nM, respectively and in the blood, IGFBP-3 is a major party of binding protein to IGF (Baxter, R. C., in Modern Concepts of Insulin-Like Growth Factors (Spencer, E. M., ed) pp371, Elsevier Science Publishing Co., New York-Amsterdam (1991)). When the serum of the normal volunteer is subjected to gel filtration under neutral conditions and the eluting position of IGF is confirmed, most of IGFs are eluted in the vicinity of 150 kDa and exist as a ternary complex (Baxter, R. C., et al., Proc. Natl. Acad. Sci. USA, 86, 6898 (1989)). This ternary complex is composed of IGF-I (or IGF-II) having a molecular weight of about 7.5 KDa, IGFBP-3 of 53 KDa inert to acid and a subunit protein of 84 KDa labile to an acid (Acid Labile Subunit; which will hereinafter be abbreviated as "ALS". It is also referred to as "α-subunit"). It is presumed that only when IGF binds to IGFBP-3 which is a major binding protein in blood, ALS binds to them and they form the above-described complex of 150 KDa.

It is considered that free IGF or a binary complex of IGF and IGFBP can pass through the capillary wall, while a ternary complex of IGF, IGFBP-3 and ALS cannot pass through it (Rechler, M.M., Vitam. Horm., 47, 1(1993)). On the other hand, concerning the half-life of human IGF in blood, that of free IGF is as short as about 10 minutes, that of the complex of IGF and IGFBP is about 30 minutes and that of the ternary complex composed of IGF, IGFBP-3 and ALS is about 15 hours (Zapf, J., et al., in Modern Concepts of Insulin-Like Growth Factors (Spencer, E. M., et) pp.591, Elsevier Science Publishing Co., New York-Amsterdam (1991)).

Accordingly, it is presumed that ALS acts to IGF together with IGFBP-3 to form a ternary complex of IGF, IGFBP-3 and ALS, thereby extending the half-life of IGF in blood and suppressing the physiological activity of IGF. Also, it is presumed that IGFBP forms a binary complex of IGF and IGFBP, thereby extending the half-life of IGF in blood and suppressing or promoting the physiological activity of IGF (Baxter, R. C., et al., Prog. Growth Factor Res., 1, 49 (1989)).

There is almost no difference in ALS between the species and homology of ALS between human being and rat is 78% (Dai, J., et al., Biochem. Biophys. Res. Commun., 188, 304 (1992)). It is said that ALS alone does not bind to IGF or IGFBP-3, but in recent days, there is a report that ALS exists as a complex with IGFBP-3 in the serum of a rat (Lee, C. Y., Endocrinology, 136, 4982 (1995)).

It is known that the IGF administration to the living body does not so much elevate the free IGF concentration in blood, although free IGF mainly exhibits activity, but elevates the concentration of IGFBP-2 in blood. From this, it is considered that there exists an IGF-dependent mechanism in the living body for regulating the expression of IGFBP (Zapf, J., et al., in Modern Concepts of Insulin-Like Growth Factors (Spencer, E. M., et) pp.591, Elsevier Science Publishing Co., New York-Amsterdam (1991)).

As a variety in the regulation mechanism caused by the ternary complex of IGF, IGFBP-3 and ALS, there is an example of non-islet cell tumor hypoglycaemia exerting low blood glucose level (tumor producing IGF-II: which will hereinafter be abbreviated as "NICTH"). In the patient of NICTH, so-called big IGF-II having a sugar chain added to the E-domain site of precursor form of IGF-II predominantly exists instead of IGF-II of 7.5 kDa which exists in normal plasma. This big IGF-II forms a complex with IGFBP-3, but cannot form a ternary complex with IGFBP-3 and ALS (Baxter, R. C., in Modern Concepts of Insulin-Like Growth Factors (Spencer, E. M., ed) pp371, Elsevier Science Publishing Co., New York-Amsterdam (1991)) so that big IGF-II is considered to exert blood glucose level lowering action without being disturbed by ALS. The big IGF-II mainly exists as a complex with IGFBP-3 in blood and this complex is said to be able to pass through the capillary vessel wall (Rechler, M.M., Vitam. Horm., 47, 1 (1993)). The complex of the big IGF-II and IGFBP-3 is therefore considered to be able to reach the target site. There is also a report that when the complex of IGF-I and IGFBP-3 was administered to a hypophysectomized rat, the action of IGF-I was recognized, though weaker than the single administration of IGF-I (Zapf, J., et al., J. Clin. Invest., 95, 179 (1995)). Accordingly, the complex of IGF and IGFBP-3 existing in blood is presumed to have ability of moving to the target site and appearing the activity of IGF there.

As another variety in the regulation mechanism by the IGF-IGFBP complex, the existence of protease for IGFBP can be mentioned. When the IGFBP-3 concentration in the blood of a gravida in the latest stage of pregnancy is analyzed, a slight increase in the IGFBP-3 concentration is recognized by RIA using an anti-IGFBP-3 antibody, while decrease in the IGFBP-3 concentration is observed when measured by the western ligand blotting method using $^{125}$I-IGF. As a result of studying what caused this difference between these measuring methods, it has been elucidated that there exists protease for decomposing IGFBP-3 in the blood of the gravida (Hossenlopp, P., et al., J. Clin. Endocrinol. Metab., 71, 797 (1990); Giudice, L.C., et al., J. Clin. Endocrinol. Metab., 71, 806 (1990)). It is considered that protease existing in blood decomposes IGFBP-3, which lowers affinity between IGF and IGFBP-3, resulting in an increase in the availability of IGF.

Thus, the activity of IGF naturally existing in the living body is regulated by IGFBP and ALS. IGF administered from the outside of the body is considered to be metabolized quickly, if it is free IGF or to form a binary complex with IGFBP which has been appeared newly or a ternary complex with the IGFBP and ALS. Even if IGF or a substance having IGF-like activity is administered from the outside of the body, the appearance of its activity is controlled by the regulation mechanism of IGF by IGFBP and/or ALS in the living body.

The present inventors therefore carried out an extensive investigation. As a result, it has been found that the activity of IGF can be expressed not by administering IGF or a substance having IGF-like activity from the outside of the body but by administering a substance which can increase, in the living body, free IGF or a substance which can increase a binary IGF-IGFBP complex exhibiting IGF-like activity.

Described specifically, it has been found that the activity of IGF can be appeared by administering a substance which can convert a binary IGF-IGFBP complex or a ternary IGF-IGFBP-ALS complex to free IGF, a substance which can convert the ternary complex to the binary IGF-IGFBP complex, a substance which can liberate, from the ternary complex, IGF or the binary IGF-IGFBP complex, or a substance which can inhibit the formation of the binary IGF-IGFBP complex or the ternary IGF-IGFBP-ALS complex.

The present invention has been accomplished based on these findings and an object is to make effective use of endogenous IGF existing in a large amount in the living body.

An object of the present invention is therefore to provide a method for elevating the concentration of free IGF, which comprises converting IGF existing in the living body into free IGF having sufficient IGF activity.

Another object of the present invention is to provide a method for elevating the concentration of the IGF-IGFBP complex which has lower IGF activity than free IGF but higher IGF activity than the IGF-IGFBP-ALS complex.

Disclosure of the Invention

In one aspect of the present invention, there is thus provided a method for elevating the concentration of free IGF, which comprises converting IGF existing in the living body into free IGF having sufficient IGF activity.

This conversion into the free IGF can be carried out by:

conversion of the IGF-IGFBP complex or IGF-IGFBP-ALS complex into IGF;
liberation of IGF from the IGF-IGFBP complex or IGF-IGFBP-ALS complex; or
inhibition of the binding of IGF and IGFBP or binding of IGF, IGFBP and ALS.

In another aspect of the present invention, there is also provided a method for elevating the concentration of an IGF-IGFBP complex which has lower IGF activity than free IGF but higher IGF activity than an IGF-IGFBP-ALS complex. The conversion into the IGF-IGFBP complex can be carried out by:

conversion of the ternary IGF-IGFBP-ALS complex to the binary IGF-IGFBP complex;
liberation of the binary IGF-IGFBP complex from the ternary complex; or
inhibition of the binding of the binary IGF-IGFBP complex to ALS.

In a further aspect of the present invention, there is also provided a substance which can elevate the concentration of free IGF or the IGF-IGFBP complex in the living body, as follows.

1) A substance which coverts the binary IGF-IGFBP complex in the living body into free IGF.
2) A substance which liberates free IGF from the binary IGF-IGFBP complex in the living body.
3) A substance which inhibits the binding of IGF and IGFBP in the living body.
4) A substance which converts the ternary IGF-IGFBP-ALS complex in the living body to the binary IGF-IGFBP complex.

5) A substance which liberates the binary IGF-IGFBP complex from the ternary IGF-IGFBP-ALS complex in the living body.

6) A substance which inhibits the binding of the binary IGF-IGFBP complex in the living body to ALS.

7) A substance which converts the ternary IGF-IGFBP-ALS complex in the living body into free IGF.

8) A substance which liberates free IGF from the ternary IGF-IGFBP-ALS complex in the living body.

9) A substance which inhibits the binding of IGF, IGFBP and ALS in the living body, thereby liberating free IGF.

10) A substance which substantially binds neither to an IGF receptor nor to an insulin receptor but binds to IGFBP.

11) An IGF derivative which substantially binds neither to an IGF receptor nor to an insulin receptor but binds to IGFBP.

12) An IGF derivative which substantially binds neither to an IGF receptor nor to an insulin receptor but binds to IGFBP, and has an amino acid sequence similar to IGF except for the addition, depletion or substitution of one or more than one amino acid residue.

13) An IGF derivative which substantially binds neither to an IGF receptor nor to an insulin receptor but binds to IGFBP, and has an amino acid sequence similar to human IGF-II except that each of the 27-th tyrosine residue and 43-rd valine residue has been substituted with a leucine residue.

14) An anti-IGFBP antibody which substantially binds neither to an IGF receptor nor to an insulin receptor but binds to IGFBP.

15) An anti-IGFBP antibody which substantially binds neither to an IGF-I receptor nor to an insulin receptor but binds to IGFBP-3.

In a further aspect of the present invention, there is also provided a medicament comprising such a substance.

In a still further aspect of the present invention, there is also provided a screening method for searching such a substance.

Brief Description of the Drawings

FIG. 1 illustrates the restriction map of plasmid BP-3 up/pUC19/Δ in which rat IGFBP-3 gene 5' end region is cloned.

FIG. 2 illustrates the restriction map of plasmid BP-3 down/pTV119N in which rat IGFBP-3 gene 3' end region is cloned.

FIG. 3 illustrates the restriction map of plasmid BP-3/pTV119N in which rat IGFBP-3 is cloned.

FIG. 4 illustrates the restriction map of plasmid RSV-LTR/rat IGFBP-3/SV2-Term/SV40-Pro/neo/SV2-Term/+Amp which is a rat IGFBP-3 expression vector in animal cells.

FIG. 5 illustrates the restriction map of secretory expression vector used for the preparation of an expression vector in E. coli.

FIG. 6 illustrates the restriction map of a plasmid having a rat IGFBP-3 gene fragment from which signal sequence has been removed.

FIG. 7 illustrates the restriction map of a plasmid which is a rat IGFBP-3 secretory expression vector in E. coli.

FIG. 8 illustrates the results of the binding test in Example 10, more specifically, affinity of IGF-I and IGF-II for rat IGFBP-3 (RBP-3 E. coli) and affinity of RBP-3 E. coli for IGF-II.

FIGS. 9A and 9B illustrate the inhibitory activity of various compounds (Table 1) against the binding of IGF-I and IGFBP-3 (Example 11).

FIG. 10 illustrates the bound amount of IGFBP-3 to immobilized RALS in the presence of IGF-I or IGF-II, indicating the capacity of each of IGF-I and IGF-II to form the complex of IGF, IGFBP-3 and ALS.

FIG. 11 illustrates the results of Example 13, indicating the affinity of ALS for the complex of IGF and IGFBP-3.

FIG. 12 illustrates the results of Example 14, indicating the affinity of ALS for IGFBP-3.

FIG. 13 illustrates the results of the IGF-I receptor binding assay performed in Example 15 by using $^{125}$I-IGF-I and IGF-I receptor.

FIG. 14 illustrates the results of the insulin receptor binding assay performed in Example 15 by using $^{125}$I-insulin and insulin receptor.

FIG. 15 illustrates the inhibition of the binding of IGF and IGFBP-3 by a human IGF-II derivative, [Leu27, Leu43]rIGF-II (Example 16).

FIG. 16 illustrates the inhibition of the binding of IGF and IGFBP-3 by an anti-IGFBP-3 antibody (Example 17).

FIG. 17 illustrates the total triglyceride concentration in the blood of SD rats, 6 and 24 hours after the administration of IGF-I, IGF-II, IGF-II derivative and anti-IGFBP-3 antibody (Example 18-1).

FIG. 18 illustrates the total cholesterol concentration in the blood of SD rats, 6 and 24 hours after the administration of IGF-I, IGF-II, IGF-II derivative and anti-IGFBP-3 antibody (Example 18-1).

FIG. 19 illustrates the total cholesterol concentration in the blood of insulin-resistant model rats, 6 hours after the administration of IGF-I and anti-IGFBP-3 antibody (Example 18-2).

FIG. 20 illustrates the total triglyceride concentration in the blood of insulin-resistant model rats, 6 hours after the administration of IGF-I and anti-IGFBP-3 antibody (Example 18-2).

FIG. 21 illustrates the free fatty acid concentration in the blood of insulin-resistant model rats, 6 hours after the administration of IGF-I and anti-IGFBP-3 antibody (Example 18-2).

FIG. 22 illustrates the free IGF-I concentration in the blood of SD rats, 1 and 6 hours after the administration of anti-IGFBP-3 antibody (Example 18-3).

## Best Modes for Carrying Out the Invention

First, a method for elevating the concentration of free IGF and a method for elevating a binary IGF-IGFBP complex according to the present invention will be described.

The method for elevating the concentration of free IGF according to the present invention is attained not by administering IGF or a substance having IGF-like activity from the outside of the living body, but by making use of endogenous free IGF or IGF existing in the binary IGF-IGFBP complex and/or ternary IGF-IGFBP-ALS complex existing originally in the living body. The term "living body" as used herein means the blood or the tissue of an organ such as liver or kidney of human beings or mammals other than human beings such as cow, horse, sheep or pig. The term "free IGF" as used herein means not only IGF converted or liberated from the binary complex or ternary complex in the living body but also IGF which has not formed the binary complex with IGFBP or ternary complex with IGFBP and ALS.

In the present invention, the concentration of free IGF is elevated by:

converting, in the binary IGF-IGFBP complex, inactive IGF to active IGF,
liberating IGF from the binary IGF-IGFBP complex,
inhibiting the binding of IGF and IGFBP,
converting, in the ternary IGF-IGFBP-ALS complex, inactive IGF to active IGF,
liberating IGF from the ternary IGF-IGFBP-ALS complex,
inhibiting the binding of IGF to IGFBP and ALS, or the like.

It is also possible to elevate the concentration of free IGF by:

converting the ternary IGF-IGFBP-ALS complex to the binary IGF-IGFBP complex,
liberating the binary IGF-IGFBP complex from the ternary IGF-IGFBP-ALS complex,
inhibiting the binding of the binary IGF-IGFBP complex to ALS, or the like, thereby increasing the concentration of the binary IGF-IGFBP complex first and then:
converting the binary IGF-IGFBP complex to IGF,
liberating IGF from the binary IGF-IGFBP complex, or the like. Since the method according to the present invention makes it possible to increase the concentration of free IGF, thereby causing IGF action to be appeared, it also serves as a method for the prevention/treatment of diseases which can be prevented and/or treated by the action of IGF.

In the present invention, the concentration of the binary IGF-IGFBP complex is elevated by:

converting the ternary IGF-IGFBP-ALS complex into the binary IGF-IGFBP complex,
liberating the binary IGF-IGFBP complex from the ternary IGF-IGFBP-ALS complex,
inhibiting the binding of the binary IGF-IGFBP complex to ALS, or the like. Accordingly, the method according to the present invention also serves as a method for the prevention/treatment of the diseases which can be prevented and/or treated by the IGF-like activity exhibited by the binary IGF-IGFBP complex.

A description will next be made of the substances according to the present invention.

Different from IGF or a substance having IGF-like activity which appeares its activity by binding to an insulin receptor and/or IGF receptor, the substance according to the present invention substantially binds neither to an insulin receptor nor to an IGF receptor.

The substance of the present invention which converts the binary IGF-IGFBP complex into IGF means a substance which acts on the binary complex, thereby converting it into IGF. The administration of a substance which converts the complex into IGF therefore increases free IGF.

The substance of the present invention which liberates IGF from the binary IGF-IGFBP complex means a substance which acts on the binary complex, thereby liberating IGF. The administration of a substance which causes liberation therefore increases free IGF.

The substance of the present invention which inhibits the binding of IGF and IGFBP means a substance which inhibits the formation of the binary IGF-IGFBP complex. The administration of a substance which inhibits the binding therefore increases free IGF.

The substance of the present invention which converts the ternary IGF-IGFBP-ALS complex into the binary IGF-IGFBP complex means a substance which acts on the ternary complex, thereby converting it into the binary IGF-IGFBP complex. The administration of a substance which converts into the binary IGF-IGFBP complex therefore increases the binary IGF-IGFBP complex.

The substance of the present invention which liberates the binary IGF-IGFBP complex from the ternary IGF-IGFBP-ALS complex means a substance which acts on the ternary complex, thereby liberating therefrom the binary IGF-IGFBP complex. The administration of a substance which liberates the binary IGF-IGFBP complex therefore increases the binary IGF-IGFBP complex.

The substance of the present invention which inhibits the binding of the binary IGF-IGFBP complex to ALS means a substance which inhibits the formation of the ternary IGF-IGFBP-ALS complex. The administration of a substance which inhibits the binding therefore increases the binary IGF-IGFBP complex.

The substance of the present invention which converts the ternary IGF-IGFBP-ALS complex into IGF means a substance which acts on the complex, thereby converting it into IGF. The administration of a substance which converts the ternary complex into IGF therefore increases free IGF.

The substance of the present invention which liberates IGF from the ternary IGF-IGFBP-ALS complex means a substance which acts on the ternary complex, thereby liberating IGF. The administration of a substance which causes liberation therefore increases free IGF.

The substance of the present invention which inhibits the binding of IGF, IGFBP and ALS means a substance which inhibits the formation of the ternary IGF-IGFBP-ALS complex, thereby increasing free IGF. The administration of a substance which inhibits the binding therefore increases free IGF.

It should be noted that in the present invention, the binary IGF-IGFBP complex and ternary IGF-IGFBP-ALS complex mean those formed by static interaction, hydrogen bonding, hydrophobic interaction or the like. They include any kinds of complexes without particular limitation on the kind of IGF, IGFBP or the like. Examples include a binary complex of IGF-I and IGFBP-3, a ternary complex of IGF-I, IGFBP-3 and ALS.

When the substance of the present invention is administered, free IGF or a binary IGF-IGFBP complex increases in a living body and IGF action is appeared so that it is useful as a medicament and moreover, useful as a preventive/remedy for the diseases which can be prevented and/or treated by IGF.

Accordingly, the substance of the present invention can be a preventive and/or remedy for, for example, diabetes mellitus, diabetic neuropathy, amyotrophic lateral sclerosis, osteoporosis and the like.

In some diseases, the kind of the substance of the present invention can be selected in consideration of the tissue specific expression of IGFBP and/or tissue specific action of IGF. Described specifically, a substance which is selective to an organ such as muscle or bone and is allowed to exhibit the effect of IGF specific to the tissue by adjusting the topical concentration of free IGF can be prepared.

The substances of the present invention each has a function as described above. No particular limitation is imposed on the substance of the present invention. Examples include substances produced by organisms such as miroorganisms, plants and animals, substances produced by the cells or tissue cultures of plants or animals, extracts from organisms and chemically synthesized compounds. They may be used either singly or in combination.

The substances of the present invention embrace insulin-like growth factor derivatives and anti-insulin-like growth factor-binding protein antibodies. The term "insulin-like growth factor derivatives" means derivatives of an insulin-like growth factor. Examples include those synthesized by subjecting insulin-like growth factors to chemical modification or the like and those having an amino acid sequence similar to an insulin-like growth factor except for the addition, depletion or substitution of one or more than one amino acid residue according to the genetic engineering technique. The addition, depletion or substitution of an amino acid residue can be carried out by the technique as described in the following literature (Genetic Engineering, 3, 1 (1981); Nucleic Acid Research, 10, 6487 (1982)) or the like.

Preferred examples of the insulin-like growth factor derivative of the present invention include a derivative obtained by substituting each of the 27-th tyrosine residue and 43-rd valine residue of the amino acid sequence of the human insulin-like growth factor-II with a leucine residue, respectively.

The substance of the present invention may be administered either parenterally or orally, but oral administration is preferred in consideration of convenience upon use. For administration, it may be formulated by the conventionally known method. Upon formulation, it is possible to add an appropriate amount of a conventionally known additive such as excipient, disintegrator, binder, lubricant, fluidity improver, dispersant, suspending agent, emulsifier, antiseptic or stabilizer as needed.

Examples of the dosage form for parenteral administration include ointment, plaster, suppository, injection, eye drops, nose drops, ear drops, inhalation, spirit, cataplasm, liniment and lotion. Examples of the dosage form for oral administration include elixir, capsule, granule, fine granule, pill, suspension, emulsion, powder, tablet, syrup, troche, dry syrup and lemonade.

The dose of the substance of the present invention may be investigated as needed in consideration of the administration route, diseases to be treated, the conditions of the patient and the like.

The present inventors found two kinds of the substances relating to the present invention and confirmed that the level of free IGF-I in blood is elevated and action of IGF is appeared by *in vivo* administration of them to a normal rat and a diabetes model rat.

A description will next be made of a method for screening the substance of the present invention.

The substance of the present invention can be searched by labeling any one of IGF, IGFBP and ALS so as to be directly or indirectly detectable and studying whether the binding of the labeled substance (e.g., IGF) to the other binding partner (e.g., IGFBP) is inhibited by the addition of a sample or whether the labeled substance is liberated from the complex of the labeled substance (e.g., IGF) and the other binding partner (e.g., IGFBP).

In the directly detectable labeling, the amount of the labeled substance can be detected directly by physical measurement. For example in the case of labeling with a radioisotope, the labeled substance can be detected directly by the measurement of radioactivity. Detection can also be carried out by Scintillation Proximity Assay (Cook, N. D., Drug Discovery today, $\underline{1}$, 287 (1996)) or the like method. When labeling is carried out with a coloring matter or fluorescence dye, the amount of the labeled substance can be optically detected directly.

In the indirect detectable labeling, the labeled substance itself cannot be detected physically but the amount of it can be determined by forming a directly detectable molecule (e.g., dye, fluorescence dye) in a stoichiometric amount through chemical reaction. For example, in the case of labeling with an enzyme, an enzyme substrate is added to produce detectable dye and the like after reaction in the presence of a sample (a substance to be tested) and then, they may be detected.

The screening using a radioisotope can be carried out in accordance with the ordinarily employed liquid phase method. For example, after reaction of $^{125}$I-labelled IGF ($^{125}$I-IGF) with IGFBP, $^{125}$I-IGF (bound type) existing as a complex can be separated from $^{125}$I-IGF (free type) which has not formed a complex by the ordinarily employed method such as:

1) a method of using activated charcoal (Moses, A. C., et al., Endocrinology, $\underline{104}$, 536 (1979); Binoux, M., et al., J. Clin. Endocrinol. Metab., $\underline{59}$, 453 (1984); Scott, C. D., et al., Endocrinology, $\underline{116}$, 1094 (1985); Szabo, L., et al., Biochem. Biophys. Res. Commun., $\underline{151}$, 207 (1988); Gelato, M. C., et al., J. Clin. Endocrinol. Metab., $\underline{70}$, 879 (1990); Oh, Y., et al., Biochem. J., $\underline{278}$, 249 (1991), etc.),

2) a method of making use of the property of a lectin protein which can recognize the sugar chain portion of IGFBP (Martin, J. L., et al., J. Biol. Chem., $\underline{261}$, 8754 (1986)),

3) a method of immunoprecipitation by using a primary antibody and secondary antibody (Martin, J. L., et al., J. Biol. Chem., $\underline{261}$, 8754 (1986); Baxter, R. C., et al., J. Biol. Chem., $\underline{264}$, 11843 (1989); Baxter, R. C., Biochem. J., $\underline{271}$, 773 (1990) or the like) and the like.

By utilizing the above-described methods and using $^{125}$I-IGF, $^{125}$I-IGFBP or $^{125}$I-ALS and an appropriate primary antibody thereto in combination, it is possible to evaluate the forming capacity of the binary IGF-IGFBP complex or ternary IGF-IGFBP-ALS complex.

These conventional methods are however not suited for the screening for many specimens because they are difficult in handling and dangerous, a radioisotope having a short half-life is used as a labeling substance and centrifugal operation is employed upon separation of the bound type/free type.

For the screening of many specimens at a high rate, Scintillation Proximity Assay can be employed. In addition, a solid phase method not using a radioisotope but using an enzyme as a labeling substance can be used conveniently.

The screening of the substance of the present invention which converts the binary IGF-IGFBP complex into free IGF or liberates free IGF from the binary IGF-IGFBP complex can be carried out, for example, by adding an enzyme-labeled IGF to immobilized IGFBP to form their complex, adding a test substance to cause reaction, washing and then measuring the activity of the enzyme used for labeling. Alternatively, it is possible to immobilize IGF and label IGFBP with an enzyme.

The substance of the present invention which inhibits the binding of IGF and IGFBP can be screened by simultaneously adding enzyme-labeled IGF and a test substance to an immobilized IGFBP to react them, washing and then measuring the activity of the enzyme used for labeling. Alternatively, it is possible to immobilize IGF, label IGFBP with an enzyme and measure the activity of the enzyme.

The substance which converts the ternary IGF-IGFBP-ALS complex into the binary IGF-IGFBP complex, the substance which liberates the binary IGF-IGFBP complex from the ternary IGF-IGFBP-ALS complex, the substance which converts the ternary IGF-IGFBP-ALS complex into free IGF or the substance which liberates IGF from the ternary IGF-IGFBP-ALS complex, each according to the present invention, can be screened, for example, by adding IGF and enzyme-labeled IGFBP to immobilized ALS to form their ternary complex, adding a test substance to react them, washing and then measuring the activity of the labeling enzyme. Alternatively, IGF can be labeled with an enzyme instead.

Screening may be carried out by adding enzyme-labeled ALS to an immobilized binary IGF-IGFBP complex, thereby forming their ternary complex, adding a test substance to react them, washing and then measuring the activity of the labeling enzyme.

Screening may also be carried out by adding IGFBP and enzyme-labeled ALS to immobilized IGF, thereby forming their ternary complex, adding a test substance to react them, washing and then measuring the activity of the labeling enzyme. In order to confirm to which the substance obtained by the screening belongs to the substance which converts the ternary IGF-IGFBP-ALS complex into the binary IGF-IGFBP complex, the substance which liberates the binary IGF-IGFBP complex from the ternary IGF-IGFBP-ALS complex, the substance which converts the ternary IGF-IGFBP-ALS complex into free IGF or the substance which liberates free IGF from the ternary IGF-IGFBP-ALS complex, it is possible to employ the above-described screening method for the substance which converts the binary IGF-IGFBP complex into free IGF or the substance which liberates free IGF from the binary complex.

The substance which inhibits the binding of the binary IGF-IGFBP complex to ALS or the substance which inhibits the binding of IGF, IGFBP and ALS, each according to the present invention, can be screened by simultaneously adding the binary complex of IGF and enzyme-labeled IGFBP and a test substance to immobilized ALS to react them, washing and then measuring the activity of the labeling enzyme. Alternatively, it is possible to label IGF with an enzyme, instead.

It is also possible to carry out screening by simultaneously adding enzyme-labeled ALS and a test substance to the immobilized binary complex of IGF and IGFBP to react them, washing and then measuring the activity of the labeling enzyme .

It is also possible to carry out screening by simultaneously adding IGFBP, enzyme-labeled ALS and a test substance to immobilized IGF to react them, washing and then measuring the activity of the labeling enzyme. It should be noted that it is possible to employ the above-described screening method for the substance which inhibits the binding of IGF and IGFBP in order to confirm to which the substance obtained by screening belongs to, the substance which inhibits the binding of the binary IGF-IGFBP complex to ALS or the substance which inhibits the binding of IGF, IGFBP and ALS.

The immobilization of IGF, IGFBP or ALS may be carried out in an ordinarily known manner. In other words, direct solid-phase method or indirect solid-phase method using avidin-biotin, hapten-anti-hapten antibody or the like may be adopted. Examples of the material for the solid phase include glass, plastics, mainly polystyrene, polyacrylamide and cellulose acetate. Examples of the form of the solid phase include test tube, bead, microtiter plate, disc, chip and the like formed using the above-exemplified material. In consideration of the screening of many specimens by using an appropriate apparatus at a high speed, it is preferred to use a commercially available multi-well microtiter plate.

The enzymatic activity may be measured by an ordinarily known method, after consideration of a labeling enzyme, substrate, buffer, pH, temperature etc. as needed. Examples include colorimetry, fluorescence assay and luminescence assay.

IGF, IGFBP or ALS may be labeled with an enzyme by an ordinarily known method and examples include maleimide method, periodic acid method and glutaraldehyde method.

The combination of an enzyme used for labeling and a substrate may be considered as needed. For example, when β-D-galactosidase is used as an enzyme, examples of the substrate include 2-nitrophenyl-β-D-galactoside, 4-methylumbelliferyl-β-D-galactoside and 5-bromo-4-chloro-3-indolyl-β-D-galactoside. When peroxidase is used as an enzyme, examples of the substrate include 2,2'-azino-bis(3-ethylbenzthiazoline-6-sulfonic acid), 3,3',5,5'-tetramethylbenzidine and 1,2-phenylenediamine. When alkaline phosphatase is used as an enzyme, examples of the substrate include 4-methylumbelliferyl phosphate and N-nitrophenyl phosphate.

Screening is preferably carried out in a buffer solution. As the buffer, an ordinarily employed one for the measurement of enzymatic activity may be used. Examples include sodium phosphate buffer, glycine-sodium hydroxide buffer and Tris-HCl buffer solution. The pH of the buffer may be investigated as needed, but is preferably 6.0 to 7.4 because there is a possibility of pH having an influence on the solubility of IGFBP. It is possible to add, to a buffer, a salt or surfactant. For example, when sodium chloride is added, its concentration up to 0.15 M is preferred.

Industrial Applicability

According to the method of the present invention, IGF action can be expressed or appeared not by administering IGF or a substance having IGF-like activity from the outside of the living body but by converting IGF existing in the living body into free IGF or a binary IGF-IGFBP complex.

The *in vivo* administration of the substance of the present invention elevated the concentration of free IGF and the action of IGF was recognized. Accordingly, the substance of the present invention is useful as a preventive and/or remedy for diseases (diabetes mellitus, amyotrophic lateral sclerosis, osteoporosis and the like) which can be prevented and/or treated by IGF.

The present invention will hereinafter be described more specifically by examples. It should however be borne in mind that the present invention is not limited by them.

Examples

Example 1: Preparation of Human IGF-I and IGF-II

Human IGF-I was purchased from GroPep Pty. Ltd. Human IGF-II was obtained in a similar manner to that of Sakano, et al. (Sakano, K., et al., J. Biol. Chem., 266, 20626 (1991)). Described specifically, human IGF-II was expressed in *Escherichia coli* by the gene recombination technique. The human IGF-II extracted from the cells was refolded and then, purified by chromatography on a reverse-phase HPLC column.

Example 2: Cloning of Rat IGFBP-3 Gene

The rat IGFBP-3 gene was cloned in accordance with the literature (Shimasaki, S., et al., Biochem. Biophys. Res. Commun., 165, 907 (1989)). Described specifically, PCR fragments were obtained, respectively, by amplifying the upstream region (5' end) and downstream region of the IGFBP-3 gene with a rat pancreas cDNA library as a template. As the PCR primer for cloning of the 5' end, 5'-CGCCATGCATCCCGCGCGCC-3' and 5'-ACGCCGCACGCGTCGCCTTC-3' were used. As the PCR primer for cloning of the 3' end, 5'-GCGCGGGCCCCGTGGTGCGCTGCGAACCGT-3' and 5'-TGCTGATCACGTTGTTGGC-3' were used.

The PCR fragments thus recovered were blunted, phosphorylated, and then inserted in pUC19 (SalI/Blunting/BAP), followed by cloning (5' end: BP-3 up/pUC19, 3' end: BP-3 down/pUCl9).

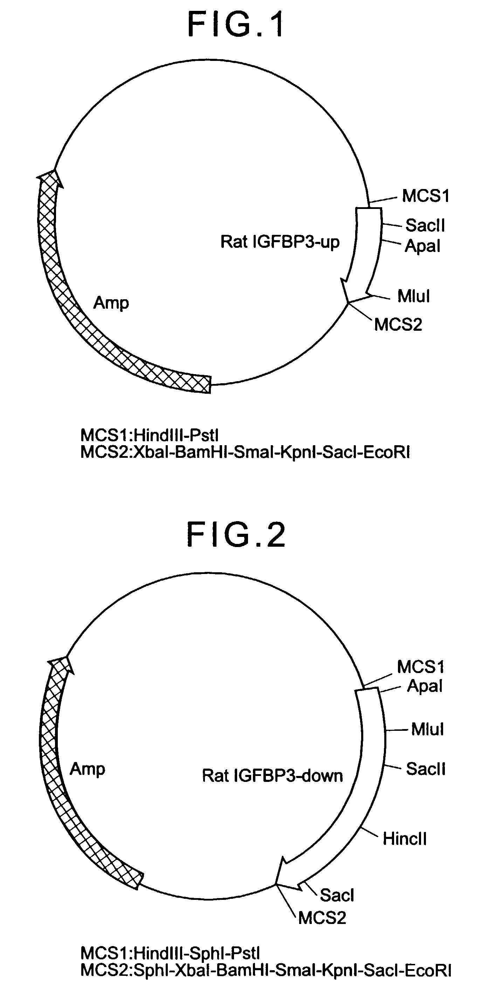

From the 5' end clone BP-3 up/pUIC19, the SphI site located upstream of a transcription initiating codon (ATG) was eliminated, whereby a plasmid BP-3 up/pUC19/ΔSphI was obtained (refer to FIG. 1). After digestion of the 3' end clone BP-3 down/pUC19 with BamHI-PstI, the fragment containing BP-3 down was recovered, blunted and inserted in pTV119N (HincII/BAP), whereby a plasmid inserted in the same direction along a lacZ promoter (BP-3 down/pTV119N: refer to FIG. 2) was obtained.

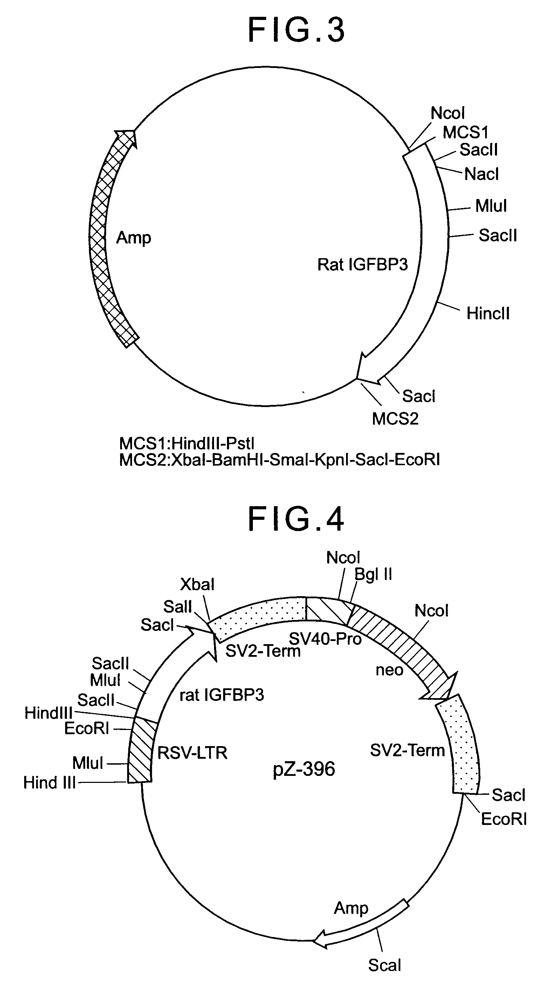

The 5' end clone (BP-3 up/pUC19/ΔSphI) was cut at MluI-HindIII and a 250 bp fragment containing BP-3 up was recovered. The 3' end clone (BP-3 down/pTV119N) was subjected to M1uI-HindIII/BAP treatment and in the resulting plasmid, the 250 bp fragment containing BP-3 up was inserted, whereby a rat IGFBP-3 clone was prepared (BP-3/pTV119N: refer to FIG. 3).

Example 3: Construction of Vector for the Expression of Rat IGFBP-3 Gene in Animal Cells

In a plasmid (Nawa, K., et al., Biochem. Biophys. Res. Commun., 171, 729 (1990)) having RSV-LTR (raus sarcoma virus long terminal repeat), the rat IGFBP-3 clone obtained above was inserted for the expression in animal cells. Described specifically, the plasmid BP-3/pTV119N was cut at XbaI-HindIII and about 900 bp fragment was obtained. The resulting fragment was inserted in an expression plasmid (SV2-Term/SV40-Pro/neo/SV2-Term/+Amp), followed by further insertion, in the HindIII site, of an about 600 bp fragment of RSV-LTR obtained by HindIII digestion (RSV-LTR/rat IGFBP-3/SV2-Term/SV40-Pro/neo/SV2-Term/+Amp) (refer to FIG. 4).

Example 4: Construction of Vector for the Expression of Rat IGFBP-3 Gene in *E. coli*

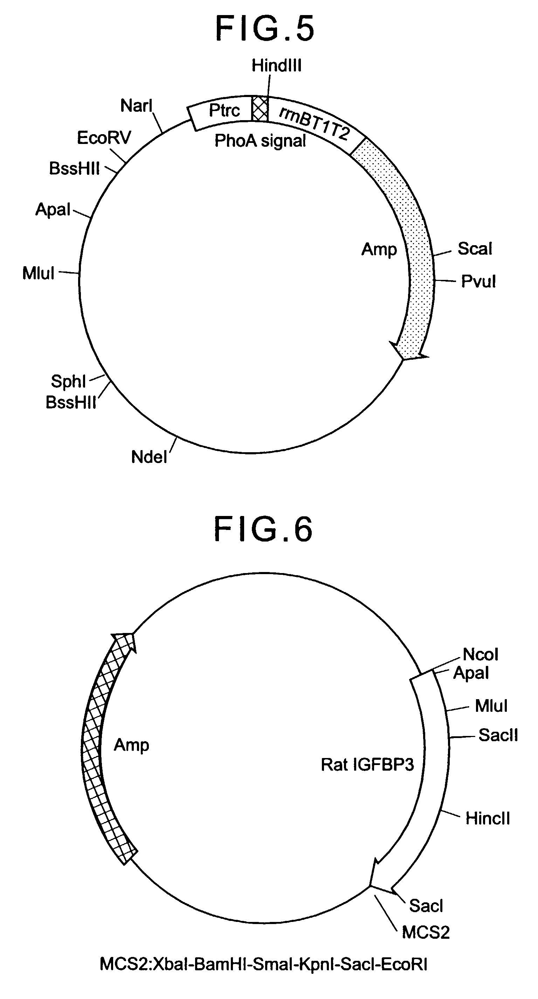

For the expression in *Escherichia coli*, a secretory expression plasmid using a PhoA signal sequence was prepared and employed. Described specifically, the PhoA signal sequence was prepared using a synthetic DNA oligomer and it was inserted in the NcoI-HindIII site of the expression plasmid pTrc99A (product of Pharmacia Biotech AB) for *E. coli* to prepare a secretory expression vector (refer to FIG. 5). It was necessary to eliminate the signal sequence from the rat IGFBP-3 precursor in order to express rat IGFBP-3 in *Escherichia coli* so that the rat IGFBP-3 gene fragment from which the signal sequence had been eliminated was recovered by cutting the plasmid BP-3/pTV119, which had been obtained in Example 2, at NaeI-XbaI.

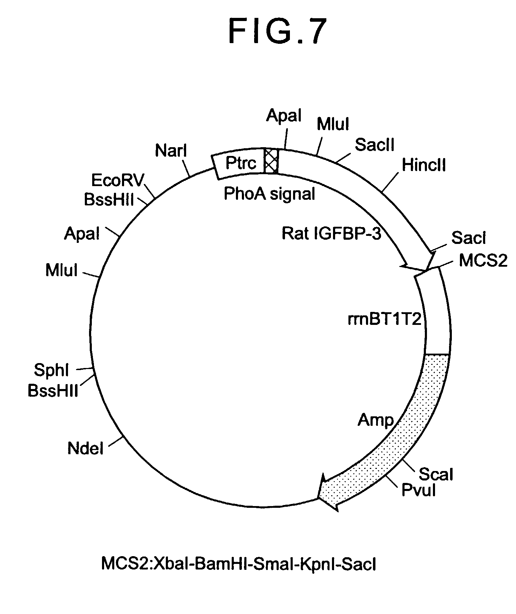

The resulting fragment was inserted in the plasmid BP-3/pTV119 previously treated with NcoI/Klenow/XbaI/BAP, whereby the plasmid shown in FIG. 6 was prepared. Since the plasmid so prepared had an NcoI site restored, it was cut at NcoI-EcoRI and then blunted with Mung Bean Nuclease, whereby the rat IGFBP-3 gene fragment from which the signal sequence had been eliminated was recovered. The resulting fragment was inserted into the secretory expression vector (HindIII/Klenow/BAP) of FIG. 5 to prepare the secretory expression plasmid of rat IGFBP-3 (refer to FIG. 7).

Example 5: Expression of Rat IGFBP-3 in Animal Cells and Purification of it

For the expression of rat IGFBP-3 in animal cells, CHO-K1 cells were employed. The plasmid of FIG. 4 for the expression in animal cells, which plasmid had been obtained in Example 3, was introduced into the CHO-K1 cells by the calcium phosphate method. Recombinant cells were cloned in DMEM/F-12 medium containing 0.4 mg/ml G-418. From the strains so expressed, elite ones were selected and cultured in T medium containing 1% ITES. The supernatant of the culture was collected and an enzyme inhibitor (2 mM benzamide / 1 mM PMSF / 100 U/ml Trasylol / 2 mM EDTA) was added thereto. The resulting mixture was filtered through a filter ("CAPSULE FILTER 0.2 $\mu$m sterilized"; product of Gelman Science), followed by the addition of a 1 M sodium acetate solution to adjust its pH to 6.0. The mixture thus adjusted was then applied to "SP-Sepharose F.F. column" (product of Pharmacia Biotech AB) equilibrated with a 10 mM sodium acetate buffer (pH 6.0) containing 0.15 M sodium chloride. After washing successively with the same buffer and a buffer containing 0.5 M sodium chloride, elution was carried out with a buffer containing 1 M sodium chloride. The eluate was diluted to two-fold and adjusted so as to finally become pH 7.0 with a sodium phosphate solution.

By using "HiTrap affinity column NHS-activated" (product of Pharmacia Biotech AB), ligand affinity column on which IGF-II had been immobilized was prepared in a conventional manner. After the column was equilibrated with a 50 mM sodium phosphate buffer (pH 7.0) containing 0.45 M sodium chloride, the SP-Sepharose F.F. elution sample obtained above was applied. After washing successively with the same buffer, a 10 mM sodium acetate buffer (pH 7.0) containing 1 M sodium chloride and water, elution was carried out with 0.5 M acetic acid. The eluate thus obtained was recovered, lyophilized, dissolved in 0.1% trifluoroacetic acid (which may hereinafter be abbreviated as "TFA") and then subjected to reverse phase HPLC.

By using a reverse phase HPLC column ("CAPCELLPAK C18 SG300", 250 x 4.6 mm I.D., Shiseido Co., Ltd.), elution was carried out with linear gradient of acetonitrile at a flow rate of 1 ml/min, whereby a rat IGFBP-3 fraction was separated. Finally, the rat IGFBP-3 (which will hereinafter be abbreviated as "RBP-3CHO") thus obtained was lyophilized and stored until use.

Example 6: Expression of Rat IGFBP-3 in *E. coli* and Purification of it

Each of the colonies of *E. coli* for the secretory expression of rat IGFBP-3 was cultured in LB medium under the conditions of 37°C and 250 rpm. When Abs=3.45 was attained, IPTG was added to give a final concentration of 0.3 mM, whereby expression was induced. From the collected cells, periplasma was recovered by the osmotic shock method (Nossal, N. G. et al., J. Biol. Chem., 241, 3055 (1966)), followed by adjustment to pH 7.2 with a phosphate buffer.

To a ligand affinity column on which IGF-II had been immobilized, the sample adjusted to pH 7.2 was applied. After the treatment in the same procedure employed for the above-described purification of RBP-3CHO (refer to Example 5), the fraction eluted with 0.5 M acetic acid was lyophilized. The powder obtained by lyophilization was dissolved in 10 ml of 0.1% TFA and the resulting solution was eluted with an acetonitrile linear gradient at a flow rate of 1 ml/min by using a reverse-phase HPLC column ("YMC-PACK PROTEIN-RP", 250 x 4.6 mm I.D., product of YMC Corporation), whereby rat IGFBP-3 was eluted and separated. Finally, the rat IGFBP-3 (which will hereinafter be abbreviated as "RBP-3E. coli") so obtained was lyophilized and stored until use.

Example 7: Purification of Rat ALS from Rat Serum

Rat ALS was purified from rat serum by the method similar to that described in the literature of Baxter, R. C. et al. (Baxter, R. C., et al., Endocrinology, 134, 848 (1994)), except that the above-described ligand affinity column of Example 5, that is, "HiTrap affinity column NHS-activated" (product of Pharmacia Biotech AB) on which IGF-II had been immobilized and the above-described RBP-3 E. coli of Example 6 were used instead, and "DEAE-5PW column" (75 x 7.5 mm I. D., TOSOH CORPORATION) was used in the final purification stage.

The final purification on the "DEAE-5PW column" was carried out by equilibrating the column with a 10 mM sodium phosphate buffer (pH 8.0) containing 50 mM sodium chloride, applying thereto the sample, eluting with a sodium chloride linear gradient in the same buffer, whereby rat ALS (which will hereinafter be abbreviated as "RALS") was fractionated. Until use, rat ALS was lyophilized and stored.

Example 8: Preparation of Human IGF-II Derivative ([Leu27, Leu43]rIGF-II)

It is known that [Leu27]rIGF-II which is human IGF-II having a leucine residue replaced for the 27-th tyrosine residue and [Leu43]rIGF-II which is human IGF-II having a leucine residue replaced for the 43-rd valine residue bind to IGF-II/cation-independent mannose-6-phosphate receptor but almost lose binding activity to an IGF-I receptor and an insulin receptor which take an important role for the appearance of biological activity, and that, in practice, they have largely lowered cell proliferation activity (Sakano, K., et al., J. Biol. Chem., 266, 20626 (1991)). Furthermore, it is known that the binding capacity of these derivatives to IGFBP-3 is almost similar to the wild type (Bach, L. A., et al., J. Biol. Chem., 268, 9246 (1993)).

With the forgoing in view, a derivative ([Leu27, Leu43]rIGF-II) having leucine residues simultaneously replaced for the 27-th tyrosine residue and 43-rd valine residue, thereby having lowered affinity for both the IGF-I receptor and insulin receptor but not losing affinity for IGFBP-3 was prepared.

The derivative ([Leu27, Leu43]rIGF-II) was prepared in accordance with the Sakano's method of preparation of human IGF-II (Sakano, K., et al., J. Biol. Chem., 266, 20626 (1991)). Described specifically, in a similar manner to that of Sakano, et al., except that DNA oligomers (5'-CTGGAAAAGAGGAAACCTCTG-3, 5'-TTCTTCGAGGATACCTC-3') used upon preparation of [Leu27]rIGF-II and [Leu43]rIGF-II were used for a synthetic DNA oligomer for mutation introduction, the derivative was prepared by using a DNA clone coding for human IGF-II as a template, introducing mutation in a conventional manner, carrying out expression in *E. coli* and then the product was purified.

Example 9: Preparation of Anti-Rat IGFBP-3 Antibody

With RBP-3CHO (rat IGFBP-3 obtained in Example 5) as an antigen, rabbits (Japanese white house rabbit, male, 5 in number) were immunized. Immunization was carried out at intervals of 2 weeks, 5 times in total. The obtained anti-serum was purified by Protein A column chromatography ("PROSEP-A", product of Bioprocessing Co.), whereby a polyclonal antibody was obtained. More specifically, the anti-serum was diluted to two-fold with PBS, adjusted to pH 7.4 and applied to Protein A column equilibrated with PBS. After washing with PBS, elution was carried out with a 0.1 M glycine-hydrochloric acid buffer (pH 3.0). Rightly after that, the eluted fraction thus obtained was concentrated by ultrafiltration. The buffer change to PBS was carried out by this ultrafiltration. Finally, five lots of polyclonal antibodies (anti-RBP-3 pAb #35, #36, #88, #89, #90) were obtained.

Example 10: Binding Test of IGF and IGFBP-3 in Solid-Phase System

The test was carried out under the below-described fundamental conditions.

A 96-well microtiter plate manufactured by Costar Corp. was employed. A 50 mM sodium phosphate buffer (pH 6.5) was employed as a basic buffer. The basic buffer was used for immobilization. The basic buffer supplemented with 0.03% Tween 20, the basic buffer supplemented with 1% BSA and the basic buffer supplemented with 0.25% BSA and 0.03% Tween 20 was employed for washing, blocking and reaction, respectively. Washing for each reaction was carried out twice with 400 µl/well. Horseradish peroxidase-labeled IGF-II (which will hereinafter be abbreviated as "HRP-IGF-II") was prepared in a conventional method by using a commercially available labeling kit (product of PIERCE Chemichal Company).

The above-described fundamental conditions are also applied to Examples 11 to 17 (each, in vitro), which will be described below, unless otherwise particularly specified.

To the microtiter plate, RBP-3 E. coli (150 ng/ml) obtained in Example 6 was added in an amount of 50 µl/well and allowed to stand overnight at 4°C for immobilization. After blocking, 50 µl/well of HRP-IGF-II (final concentration: diluted to 12,000-fold) was added, followed by reaction at 25°C for 2 hours. In the end, 100 µl/well of a solution of 2,2'-azino-bis(3-ethylbenzthiazoline-6-sulfonic acid) (which will hereinafter be abbreviated as "ABTS") (product of Kirkegaard & Perry Laboratories) was added to the well and allowed to stand at room temperature. Then, the absorbance (Abs 405 nm) was measured by a plate reader ("Vmax", product of Molecular Devices).

In the case of competitive inhibition experiment using a test specimen, HRP-IGF-II (final concentration: diluted to 12,000-fold) and each one of various test specimens (competition inhibitors) were added simultaneously to each well in total amount of 50 µl with various concentrations of inhibitors, followed by the reaction similar to the above one.

The absorbance when no inhibitor was added was designated as "$B_0$", while that when an inhibitor was added was designated as "B". The absorbance when an assay was performed without the addition of a test specimen (inhibitor) and using a non-immobilized well not containing RBP-3E. coli was designated as nonspecific bound (NSB). The bound (%) of the labeled IGF-II to the immobilized IGFBP-3 when the test specimen was added was calculated from the following equation:

$$(B - NSB) / (B_0 - NSB) \times 100$$

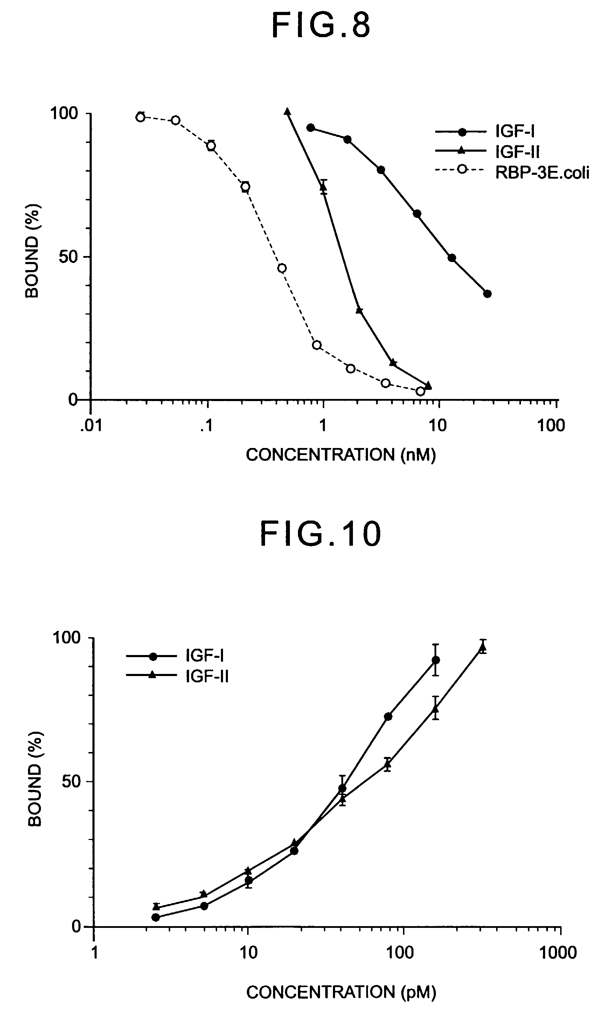

The bound was measured in triplicate by using IGF-I, IGF-II and RBP-3E. coli (IGFBP-3) as test specimens. The average value was plotted and the SD value was indicated with a bar (refer to FIG. 8).

The use of the above-described binding test makes it possible to evaluate the affinity of various IGF and IGFBP derivatives for IGFBP and moreover, to screen the substance which inhibits the binding of IGF and IGFBP.

Example 11: Experiment on the Inhibition of Various Compounds against the Binding of IGF and IBFBP-3

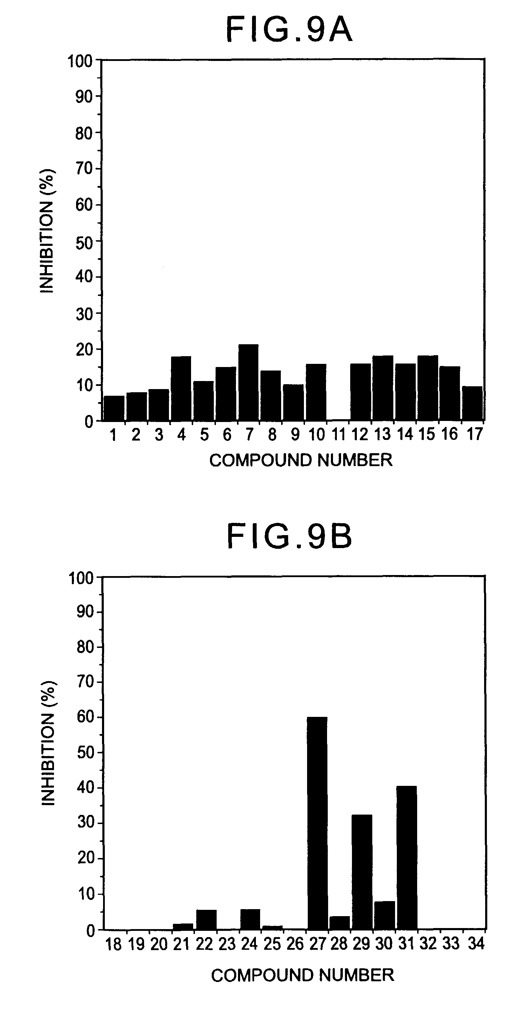

The binding inhibition experiment was carried out in accordance with Example 10, by using, as test specimens, compounds shown below in Table 1. Each compound was dissolved or suspended in the basic buffer containing 5% methanol to give a concentration of 1 mg/ml. The resulting solution or suspension was diluted with the reaction buffer to 5-fold to 200 $\mu$g/ml. The bound (%) of IGF-II to IGFBP-3 (RBP-3 E. coli) at the time when the diluted solution or suspension was added as a test specimen was measured. "100(%) - bound (%)" was designated as inhibitory activity (%) of the test specimen. Measurement was conducted twice and the average of the inhibitory activity was determined. Results are shown in FIGS. 9A and 9B. The final concentration of each compound upon measurement was 100 $\mu$g/ml. Compound 27 (Ellagic acid), Compound 29 (Aclacinomycin A) and Compound 31 (heparin) have been recognized to exhibit high inhibitory activity.

Table 1

| No. | Compound |
|---|---|
| 1 | Samarosporin |
| 2 | Hydroxy aspergillic acid |
| 3 | Kidamycin |
| 4 | Siccanin |
| 5 | Comenic acid |
| 6 | Kinetin |
| 7 | 2-Chloro-4,6-bisethylamino-5-triazine |
| 8 | Methyl hesperidine |
| 9 | Oxyperitin |
| 10 | Protionamide |
| 11 | Quercetin |
| 12 | Flavone |
| 13 | Glycyrrhizin |
| 14 | Naringenin |
| 15 | 2-Hydroxychalcone |
| 16 | N-(Methylamino)-succinamide |
| 17 | D-(+)-Catechin |
| 18 | 2-Carbethoxy-5,7-dihydroxy-4-methoxyisoflavone |
| 19 | (-) -Epicatechin |
| 20 | Betulin |
| 21 | $\alpha$-naphtoflavone |
| 22 | Curcumin |
| 23 | Tamarixetin-7-rutinoside |
| 24 | Aescin |
| 25 | Ursolsaure |
| 26 | Fisetin |

Table 1 (continued)

| No. | Compound |
|---|---|
| 27 | Ellagic acid |
| 28 | Oleanolsaure |
| 29 | Aclacinomycin A |
| 30 | Sulfonazo III |
| 31 | Heparin |
| 32 | Chondroitin sulfate |
| 33 | Vitamin B$_{12}$ |
| 34 | Vitamin B$_6$ |

Example 12: Binding Test of IGF and IGFBP-3 to ALS in Solid Phase System (1)

Under the fundamental conditions as described in Example 10, streptoavidin (1 µg/ml) was added to a microtiter plate in an amount of 50 µl/well and allowed to stand at least overnight at 4°C for immobilization. After blocking with a blocking buffer, RALS (rat ALS of Example 7: 50 ng/ml) which had been biotinated using a biotinating kit (product of Amersham International plc) according to a conventional method was added in an amount of 50 µl/well, followed by reaction at 25°C for 2 hours. To the reaction mixture, RBP-3E. coli (final concentration: 25 ng/ml) and, as a test substance, IGF-I or IGF-II of varied concentrations were added simultaneously to give a total amount of 50 µl/well and they were reacted overnight at 4°C. To the ternary complex of IGF, IGFBP-3 and ALS thus formed by the above reaction, the anti-RBP-3 pAb #35 (3 µg/ml) obtained in Example 8 was added in an amount of 50 µl/well, followed by the reaction at 25°C for 2 hours. To the reaction mixture, a labeled secondary antibody (anti-rabbit IgG, horseradish peroxidase linked whole antibody, diluted to 1000-fold, product of Amersham International plc) was added in an amount of 50 µl/well and the mixture was reacted at 25°C for 2 hours. Finally, an ABTS solution was added in an amount of 100 µl/well and the mixture was allowed to stand at room temperature for 20 minutes. The absorbance (Abs405 nm) was then measured.

The value (absorbance) when IGF-I or IGF-II is added as a test substance is designated as "B", while the value (absorbance) when 300 pM of IGF-II is added as a test substance is designated as total bound (100%). The value (absorbance) when assayed without the addition of biotinated RALS is designated as nonspecific bound (NSB). The bound (%) is calculated from the following equation:

$$(B - NSB) / (total\ bound - NSB) \times 100$$

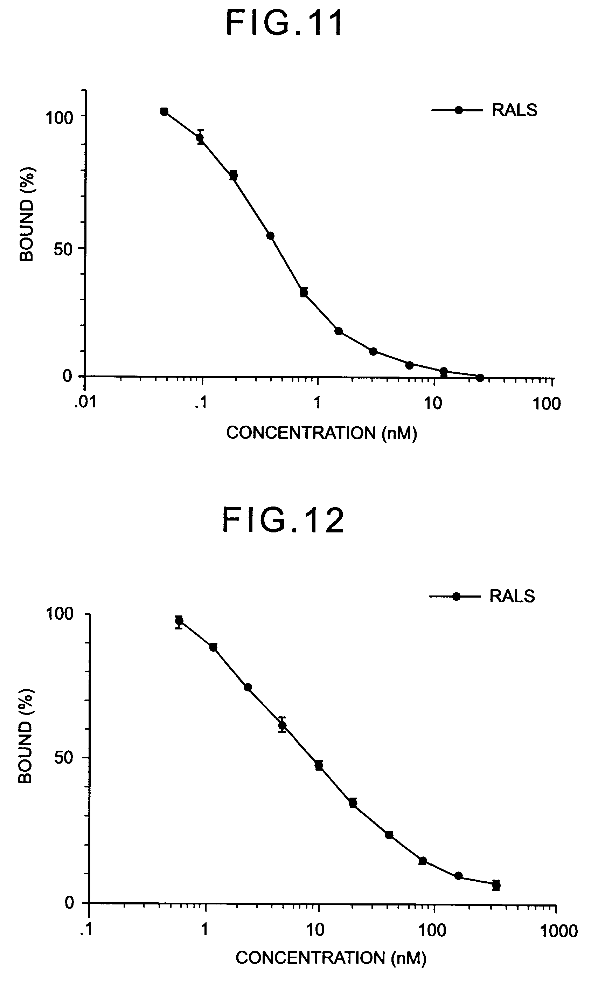

The measurement is carried out at n=3. The average of them is plotted and an SD value is indicated by a bar (refer to FIG. 10).

By adopting the above-described binding test, it is possible to evaluate the capacity of, for example, IGF or IGF derivative to form its ternary complex with IGFBP and ALS. In addition, it is possible to screen the substance which inhibits the formation of the ternary complex of IGF, IGFBP and ALS by adding the substance simultaneously (or before and after the addition) with RBP-3E. coli and IGF-I or IGF-II.

Example 13: Binding Test of IGF and IGFBP-3 to ALS in Solid-Phase System (2)

Under the fundamental conditions as described in Example 10, streptoavidin (1 µg/ml) was added to a microtiter plate in an amount of 50 µl/well and allowed to stand at least overnight at 4°C for immobilization. After blocking with a blocking buffer, the biotinated RALS (200 ng/ml) was added in an amount of 50 µl/well, followed by reaction at 25°C for 2 hours. To the reaction mixture, HRP-IGF-II (final concentration: diluted to 2000-fold) and RBP-3E. coli (final concentration: 25 ng/ml) were added simultaneously in a total amount of 50 µl/well and they were reacted at 25°C for 2 hours. In the end, after washing, an ABTS solution was added in an amount of 100 µl/well and the mixture was allowed to stand at room temperature for 20 minutes. The absorbance (Abs 405nm) was then measured.

As a competitive inhibition experiment, after the reaction of the biotinated RALS, HRP-IGF-II (final concentration: diluted to 1/2000) and RBP-3E. coli (final concentration: 25 ng/ml) and, as a competitive inhibiting substance, RALS (various concentrations) were added simultaneously in an amount of 50 µl/well in total, followed by the similar reactions.

The absorbance when assayed without the addition of biotinated RALS is designated as nonspecific bound (NSB), that assayed without an inhibiting substance is designated as "$B_0$" and that assayed with the addition of an inhibiting substance is designated as "B". The bound (%) of IGFBP-3 and IGF-II to immobilized RALS when the competitive inhibiting substance (RALS) is added is calculated from the following equation:

$$(B - NSB) / (B_0 - NSB) \times 100$$

The measurement is carried out at n=3. The average of them is plotted and an SD value is indicated by a bar (refer to FIG. 11).

By adopting the above-described binding test, it is possible to evaluate the affinity of, for example, ALS or various ALS derivatives for the complex of IGF and IGFBP-3, and in addition to screen the substance which inhibits the formation of the ternary complex of IGF, IGFBP and ALS or substance which inhibits the binding of the binary complex of IGF and IGFBP-3 to ALS.

Example 14: Binding Test of IGFBP-3 to ALS in a Solid Phase System

Under the fundamental conditions as described in Example 10, streptoavidin (1 $\mu$g/ml) was added to a microtiter plate in an amount of 50 $\mu$l/well and allowed to stand at least overnight at 4°C for immobilization. After blocking with a blocking buffer, biotinated RALS (200 ng/ml) was added in an amount of 50 $\mu$l/well, followed by reaction at 25°C for 2 hours. To the reaction mixture, RBP-3E. coli (100 ng/ml) was added in an amount of 50 $\mu$l/well and they were reacted overnight at 4°C. To the complex of IGFBP-3 and ALS formed by the above reaction. The anti-RBP-3 pAb #35 (3 $\mu$g/ml) obtained in Example 9 added in an amount of 50 $\mu$l/well, followed by reaction at 25°C for 2 hours. Then, a secondary antibody was added in an amount of 50 $\mu$l/well, followed by reaction at 25°C for 2 hours. In the end, an ABTS solution was added in an amount of 100 $\mu$l/well and the mixture was allowed to stand at room temperature for 20 minutes. The absorbance (Abs 405nm) was then measured. In the case of a competitive inhibition experiment, after the reaction of the biotinated RALS, RBP-3E. coli (final concentration: 100 ng/ml) and, as a competitive inhibiting substance, RALS (varied concentrations) were added simultaneously in a total amount of 50 $\mu$l/well, followed by the similar reactions.

The measured value when assayed without the addition of biotinated RALS is designated as nonspecific bound (NSB), that assayed without an inhibiting substance is designated as "$B_0$" and the value when assayed with the addition of an inhibiting substance is designated as "B". The bound (%) of RBP-3E. coli to immobilized RALS when the competitive inhibiting substance (RALS) is added is calculated from the following equation:

$$(B - NSB) / (B_0 - NSB) \times 100$$

The measurement is carried out at n=3. The average of them is plotted and an SD value is indicated by a bar (refer to FIG. 12).

By adopting the above-described binding test, it is possible to evaluate the affinity of, for example, ALS or ALS derivative for IGFBP-3, and in addition to screen the substance which inhibits the formation of the complex of IGFBP-3 and ALS.

Example 15: Characterization of Human IGF-II Derivative (Leu27, Leu43]rIGF-II *in vitro* (1)

How much [Leu27, Leu43]rIGF-II obtained in Example 8 lost the affinity for an IGF-I receptor or insulin receptor was evaluated, by substantially the same method of the radioreceptor assay using an IGF-I receptor or insulin receptor purified from the human placenta (Le Bon, T. R., et al., J. Biol. Chem., 261, 7685 (1986); Fujita-Yamaguchi, Y., et al., J. Biol. Chem., 258, 5045 (1983)).

First, $^{125}$I-IGF-I (or $^{125}$I-insulin) (product of Amersham International plc) in an amount of $2 \times 10^4$ cpm and each receptor were incubated and an amount of the receptor, which was to be added to the assay system, permitting 50% binding was determined. To the receptor and $^{125}$I-IGF-I or $^{125}$I-insulin ($2 \times 10^4$ cpm), [Leu27, Leu43]rIGF-II was added at each concentration. With a 50 mM Tris-HCl buffer (pH 7.4) containing 0.1% BSA and 0.075% Triton X-100, the reaction mixture was filled up to 300 $\mu$l, followed by reaction overnight at 4°C. After the reaction, 75 $\mu$l of human $\gamma$-globulin (4 mg/ml) and 375 $\mu$l of 20% PEG 6000 (pH 7.0) were added simultaneously, followed by reaction at 4°C for 1 hour. The reaction mixture was then centrifuged (3000 rpm x 30 mm, 4°C). The supernatant thus obtained was removed and $\gamma$-count (CPM) contained in the precipitate was measured.

For comparison, the IGF-I receptor assay was operated in a similar manner except for the addition of IGF-I or IGF-II of each concentration as a test specimen instead of [Leu27, Leu43]rIGF-II. The insulin receptor assay was operated in a similar manner except for the use of insulin or IGF-II of each concentration as a test substance instead of [Leu27, Leu43]rIGF-II.

The value when assayed without the addition of a receptor is designated as nonspecific bound (NSB), the value when assayed without a test substance is designated as "$B_0$" and the value when assayed with the addition of a test substance is designated as "B". The bound (%) to a receptor is calculated from the following equation:

$$(B - NSB) / (B_0 - NSB) \times 100$$

The measurement is carried out at n=3. The average of them is plotted and an SD value is indicated by a bar.

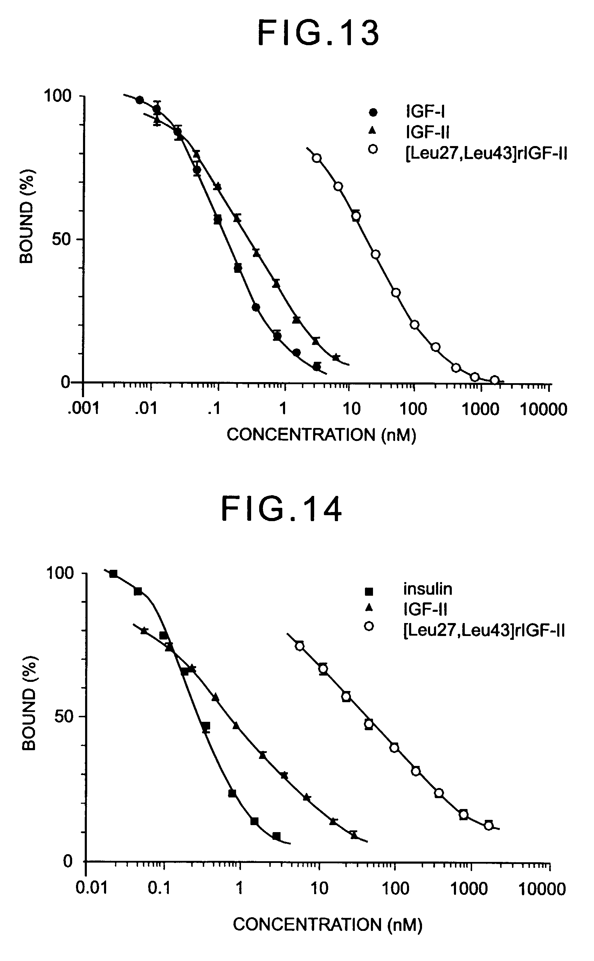

FIG. 13 illustrates the results of IGF-I receptor binding assay using $^{125}$I-IGF-I and IGF-I receptor. It has been confirmed that the affinity of IGF-II for an IGF-I receptor was recognized to be similar to that of IGF-I, while the affinity of [Leu27, Leu43]rIGF-II for the IGF-I receptor decreased even to 1 to 2% of that of IGF-I.

FIG. 14 illustrates the results of insulin receptor binding assay using $^{125}$I-insulin and insulin receptor. It has been confirmed that the affinity of IGF-II for an insulin receptor was recognized to be similar to that of insulin, while the affinity of [Leu27, Leu43]rIGF-II for the insulin receptor decreased even to 1 to 2% of that of insulin.

Example 16: Characterization of Human IGF-II Derivative ([Leu27, Leu43]rIGF-II) *in vitro* (2)

It was evaluated in accordance with Example 9 whether [Leu27, Leu43]rIGF-II inhibits the binding of IGF and IGFBP-3.

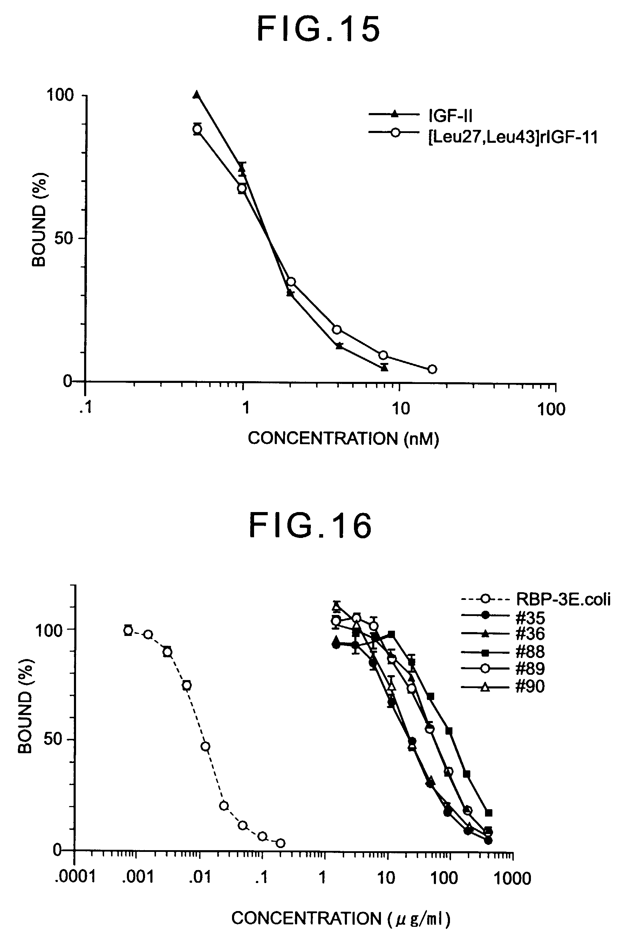

As a result, it has been confirmed that [Leu27, Leu43]rIGF-II inhibited the binding of labeled IGF-II and immobilized IGFBP-3 at a concentration almost the same with that of IGF-II (refer to FIG. 15).

Example 17: Characterization of Anti-IGFBP-3 Antibody

It was evaluated in accordance with Example 10 how much the anti-IGFBP-3 antibody (anti-RBP-3 pAb) obtained in Example 9 exhibited the binding of IGF and IGFBP-3. The measurement was carried out in utterly the same manner to Example 10, except that the anti-IGFBP-3 antibody was used as a test substance (competitive inhibiting substance).

As a result, the antibodies #35 and #90 exhibited the strongest binding inhibition activity, 0.2632% as a molar ratio (2% as a weight ratio) when the activity of RBP-3E. coli (IGFBP-3) was set at 100%. The antibodies #36 and #89 exhibited almost the same activity, 0.0875% as a molar ratio, while the antibody #88 exhibited a little lower activity, 0.0525% as a molar ratio. It has been confirmed that any one of the antibodies inhibited the binding of IGF and IGFBP-3 (refer to FIG. 16).

Example 18: Characterization of Human IGF-II Derivative and Anti-IGFBP-3 Antibody *in vivo*

It is necessary to study whether the method or substance of the present invention can practically exhibits *in vivo* effectiveness of IGF so that evaluation was carried out on the following two points:

(1) whether the administration of the substance of the present invention brought about effects similar to the administration of IGF, and
(2) whether the administration of the substance of the present invention increased the concentration of free IGF-I, which is a major party of IGF in the blood of a rat.

As effects recognizable upon IGF administration, lowering in the blood lipid level is known. Specific effects are:

(a) lowering in the level of free fatty acid (Turkalj. I., et al., J. Clin. Endocrinol. Metab., 75, 1186 (1992)),
(b) lowering in the level of triglyceride (Turkalj. I., et al., J. Clin. Endocrinol. Metab., 75, 1186 (1992); Zenobi, P. D., et al., J. Clin. Invest., 90, 2234 (1992)),
(c) lowering in the total cholesterol due to the lowering in the level of LDL-cholesterol (Kazumi, T., Metabolism, 35, 1024 (1986); Zenobi, P. D., et al., Diabetologia, 36, 465 (1993)), and the like.

It was then investigated whether such effects can be brought about by the administration of the binding inhibitory substances obtained in Example 8 and Example 9.

Example 18-1: Evaluation of Blood Lipid Level (1)

To a group of SD rats (n=3, 8 weeks old, male, average weight: 300 g, purchased from Charles River Japan), [Leu27, Leu43]rIGF-II (1,000 µg/kg) obtained in Example 8 was subcutaneously administered under unfasted conditions. To another SD rat group, anti-RBP-3 pAb #35 (20 mg/head) obtained in Example 9 was intraperitoneally administered. AS a negative control group, physiological saline (500 μl/head) was subcutaneously administered, while as a positive control group, IGF-I (50 μg/kg, 200 μg/kg) was subcutaneously administered. About 500 μl of the blood was sequentially collected into an EDTA-coated glass tube from the caudal vein. Thirty minutes after the collection, the blood was centrifuged (3,500 rpm x 12 min, 25°C). The supernatant plasma was recovered and then stored at -80°C until the beginning of the assay. The total cholesterol and total triglyceride concentrations of the plasma were measured in accordance with the standard measuring method by a "Hitachi 715 model" automatic analyzer based on the enzyme method using "Autocera CH02 and TG2" (each, manufactured by Daiichi Pure Chemicals Co., Ltd.).

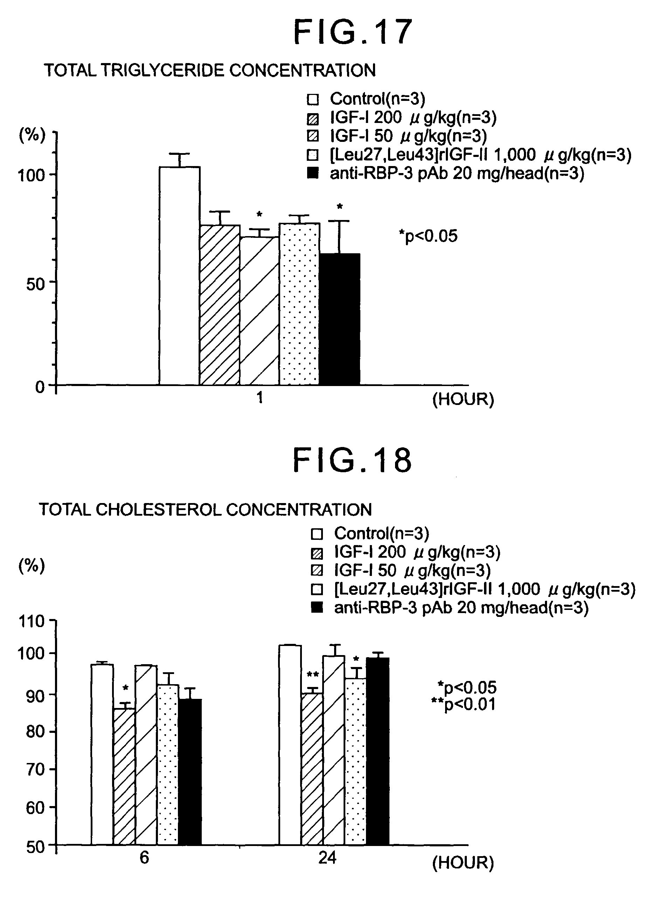

As a result, compared with the negative control group, the total glyceride concentration 6 hours after the administration showed a significant decrease by the administration of IGF-I (50 μg/kg) and anti-RBP-3 pAb #35, and a lowering tendency was also recognized in the administration of [Leu27, Leu43]rIGF-II (refer to FIG. 17). Compared with the negative control group, the total cholesterol concentration showed a significant decrease 6 and 24 hours after the administration of IGF-I (200 μg/kg). It showed a significant decrease 24 hours after the administration of [Leu27, Leu43]rIGF-II. The lowering tendency was also recognized even after the administration of anti-RBP-3 pAb #35 (refer to FIG. 18).

From the above results, it has been confirmed that the administration of such a binding inhibitory substance brought about lipid level lowering action similar to the administration of IGF-I.

In FIGS. 17 and 18, shown are the concentrations (%) of the total triglyceride and total cholesterol after administration when the concentration before administration (0 hour) was set at 100% and SEM (Standard Error Means) was indicated by a bar.

Example 18-2: Evaluation of Blood Lipid Level (2)

To a group of Zucker fatty rats (n=7 to 9, 6 to 11 weeks old, male, purchased from Tokyo Jikken Dobutsusha) known as an insulin resistant model, anti-RBP-3 pAb #36 (40 mg/head, n=5) obtained in Example 9 was subcutaneously administered after fasted for 20 hours from the day before the beginning of the administration. As a negative control group, physiological saline (500 μl/head) was subcutaneously administered, while as a positive control group, IGF-I (300 μg/kg, n=8), or IGF-II (1,200 μg/kg, n=8) or (600 μg/kg, n=8) was subcutaneously administered. From each group, blood was collected 6 hours after the administration. The total cholesterol and total triglyceride concentrations of the plasma were measured in accordance with Example 18-1. Based on the enzyme method using "NEFAC-test Wako" (product of Wako Pure Chemicals), the concentration of free fatty acid in plasma was determined by reacting in a 96-well microtiter plate (product of Costar) with a 1/20 scale in the standard operating method and then measuring the absorbance (Abs 550 nm) by a plate reader ("Vmax", product of Molecular Devices Ltd.).

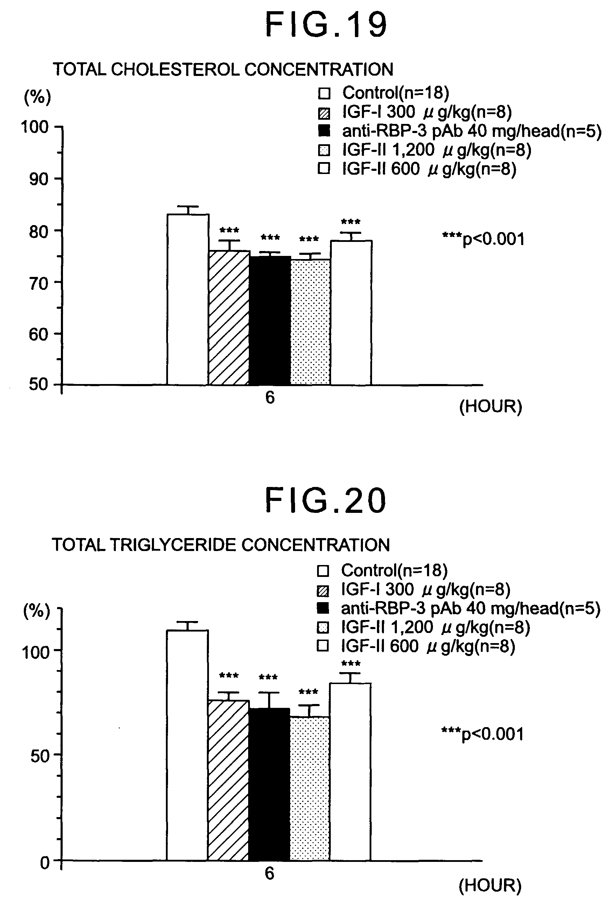

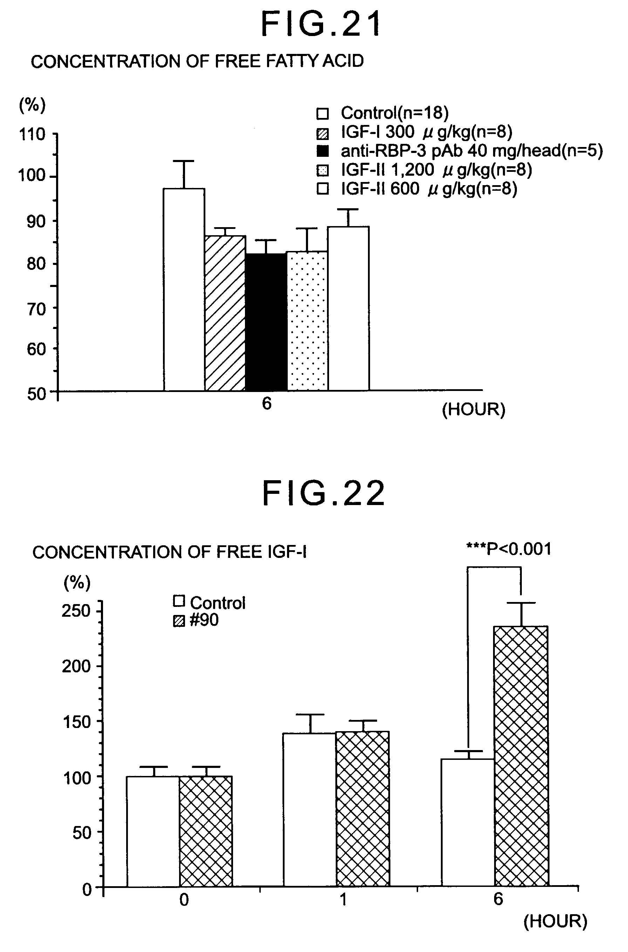

As a result, compared with the negative control group, the total cholesterol concentration showed a significant decrease in all the administration groups 6 hours after the administration (refer to FIG. 19). Also in the total glyceride concentration, a significant decrease was observed in all the administration groups 6 hours after the administration (refer to FIG. 20). A similar tendency was also observed in the concentration change of free fatty acid (refer to FIG. 21).

From the above results, it has been confirmed that the administration of such a binding inhibitory antibody (that is, an anti IGFBP-3 antibody which inhibits the binding of IGF and IGFBP-3) brought about lipid level lowering action similar to the administration of IGF-I.

In FIGS. 18, 19 and 20, shown are the concentrations (%) of total cholesterol and total triglyceride after the administration when the respective concentrations before administration (0 hour) were set at 100% and SEM (Standard Error Means) was indicated by a bar.

Example 18-3: Evaluation of Free IGF-I Level in Blood

To a group of SD rats (n=7, 8 weeks old, male, average weight: 300 g, purchased from Charles River Japan), the anti-RBP-3 pAb #90 (40 mg/head) obtained in Example 9 was intraperitoneally administered under unfasted conditions. As the negative control group, physiological saline (500 μl/head) was subcutaneously administered. The sequential blood collection was carried out in accordance with Example 18-1.

Free IGF-I in plasma and total IGF-I in plasma were prepared, respectively by the reverse-phase cartridge ("Sep-Pak C18 cartridge") method (Hizuka, N., et al., Growth Regulation, 1, 51 (1991)) and the formic acid/acetone extraction method (Bowsher, R. R., et al., Endocrinology, 128, 805 (1991)).

The rat IGF-I radioimmunoassay was carried out according to the conventional method (Moses, A. C., et al., Eur. J. Biochem., 103, 401 (1980); Daughaday, W. H., Methods Enzymol., 146, 248 (1987)). $^{125}$I-IGF-I and somatomedin-C antiserum (anti IGF-I antibody), each produced by Eiken Chemical Ltd., were used and rat IGF-I produced by Gropep Pty Ltd was used as an authentic standard. First, $^{125}$I-IGF-I (7.77 x 10[3] cpm/100 μl), about 50 μl of the antiserum and the standard or test specimen were mixed. The resulting mixture was adjusted to 300 μl with an assay buffer (25 mM sodium phosphate buffer (pH 7.5) containing 0.25% BSA, 0.05% Tween 20 and 0.1% NaN₃) and reacted overnight at 4°C. To the reaction mixture, 75 μl of 4 mg/ml human γ-globulin and 375 μl of 25% PEG 6000 were added and the mixture was reacted at 4°C for 1 hour, followed by centrifugation (3,000 rpm x 20 min, 4 °C). The centrifugal supernatant was removed and γ-count (CPM) contained in the precipitate was measured.

As a result, compared with the negative control group, the concentration ratio of the free IGF-I to the total IGF-I showed a significant increase 6 hours after the administration of anti-RBP-3 pAb#90 and it was confirmed that the free IGF-I concentration increased by the administration of the binding inhibitory antibody (refer to FIG. 22).

In FIG. 22, the ratios 1 and 6 hours after the administration were shown when the ratio of the free IGF-I to the total amount of IGF-I (free/total) before the administration (0 hour) is set at 100% and SEM value was indicated by a bar.

## Claims

1. A method for elevating the concentration of a free insulin-like growth factor, which comprises converting a binary complex composed of an insulin-like growth factor and an insulin-like growth factor binding protein in living bodies into the insulin-like growth factor.

2. A method for elevating the concentration of a free insulin-like growth factor, which comprises releasing, from a binary complex composed of an insulin-like growth factor and an insulin-like growth factor binding protein in living bodies, the insulin-like growth factor.

3. A method for elevating the concentration of a free insulin-like growth factor, which comprises inhibiting the binding of an insulin-like growth factor and an insulin-like growth factor binding protein in living bodies.

4. A method for elevating the concentration of a binary complex composed of an insulin-like growth factor and an insulin-like growth factor binding protein, which comprises converting a ternary complex composed of the insulin-like growth factor, the insulin-like growth factor binding protein and an acid labile subunit in living bodies into the binary complex composed of the insulin-like growth factor and insulin-like growth factor binding protein.

5. A method for elevating the concentration of a binary complex composed of an insulin-like growth factor and an insulin-like growth factor binding protein, which comprises releasing, from a ternary complex composed of the insulin-like growth factor, the insulin-like growth factor binding protein and an acid labile subunit in living bodies, the binary complex composed of the insulin-like growth factor and insulin-like growth factor binding protein.

6. A method for elevating the concentration of a binary complex composed of an insulin-like growth factor and an insulin-like growth factor binding protein, which comprises inhibiting the binding of the binary complex of the insulin-like growth factor and the insulin-like growth factor binding protein in living bodies to an acid labile subunit.

7. A method for elevating the concentration of a free insulin-like growth factor, which comprises converting a ternary complex composed of an insulin-like growth factor, insulin-like growth factor binding protein and an acid labile subunit in living bodies into the insulin-like growth factor.

8. A method for elevating the concentration of a free insulin-like growth factor, which comprises releasing, from a ternary complex composed of an insulin-like growth factor, insulin-like growth factor binding protein and an acid labile subunit in living bodies, the insulin-like growth factor.

9. A method for elevating the concentration of a free insulin-like growth factor, which comprises inhibiting the binding of an insulin-like growth factor, insulin-like growth factor binding protein and an acid labile subunit in living bodies.

10. A substance which converts a binary complex composed of an insulin-like growth factor and an insulin-like growth factor binding protein in living bodies into a free insulin-like growth factor.

11. A substance which releases a free insulin-like growth factor from a binary complex composed of an insulin-like growth factor and an insulin-like growth factor binding protein in living bodies.

12. A substance which inhibits the binding of an insulin-like growth factor and an insulin-like growth factor binding protein in living bodies.

13. A substance which converts a ternary complex composed of an insulin-like growth factor, an insulin-like growth factor binding protein and an acid labile subunit in living bodies into a binary complex composed of the insulin-like growth factor and insulin-like growth factor binding protein.

**14.** A substance which releases, from a ternary complex composed of an insulin-like growth factor, an insulin-like growth factor binding protein and an acid labile subunit in living bodies, a binary complex composed of the insulin-like growth factor and insulin-like growth factor binding protein.

**15.** A substance which inhibits the binding of a complex of an insulin-like growth factor and an insulin-like growth factor binding protein in living bodies to an acid labile subunit.

**16.** A substance which converts a ternary complex composed of an insulin-like growth factor, an insulin-like growth factor binding protein and an acid labile subunit in living bodies into a free insulin-like growth factor.

**17.** A substance which releases, from a ternary complex composed of an insulin-like growth factor, insulin-like growth factor binding protein and an acid labile subunit in living bodies, a free insulin-like growth factor.

**18.** A substance which releases a free insulin-like growth factor by inhibiting the binding of an insulin-like growth factor, an insulin-like growth factor binding protein and an acid labile subunit in living bodies.

**19.** A substance which substantially binds neither to an insulin-like growth factor receptor nor to an insulin receptor but binds to an insulin-like growth factor binding protein.

**20.** An insulin-like growth factor derivative, which substantially binds neither to an insulin-like growth factor receptor nor to an insulin receptor but binds to an insulin-like growth factor binding protein.

**21.** An insulin-like growth factor derivative, which substantially binds neither to an insulin-like growth factor receptor nor to an insulin receptor but binds to an insulin-like growth factor binding protein, and has an amino acid sequence similar to an insulin like growth factor except for the addition, depletion or substitution of one or more than one amino acid residue.

**22.** An insulin-like growth factor derivative, which substantially binds neither to an insulin-like growth factor receptor nor to an insulin receptor but binds to an insulin-like growth factor binding protein, and has an amino acid sequence similar to human insulin-like growth factor-II except that the 27-th tyrosine residue and 43-rd valine residue each has been substituted with a leucine residue.

**23.** An anti-insulin-like growth factor binding protein antibody, which substantially binds neither to an insulin-like growth factor receptor nor to an insulin receptor but binds to an insulin-like growth factor binding protein.

**24.** An anti-insulin-like growth factor binding protein antibody, which substantially binds neither to an insulin-like growth factor receptor nor to an insulin receptor but binds to an insulin-like growth factor binding protein-3.

**25.** A medicament, which comprises a substance as recited in any one of claims 10 to 19.

**26.** A medicament, which comprises an insulin-like growth factor derivative as recited in any one of claims 20 to 22.

**27.** A medicament, which comprises an anti-insulin-like growth factor binding protein antibody as recited in claim 23 or 24.

**28.** A screening method of a substance as recited in any one of claims 10 to 19, which comprises labeling any one of the insulin-like growth factor, insulin-like growth factor binding protein and acid labile subunit so as to be directly or indirectly detectable.

# FIG.1

MCS1:HindIII-PstI
MCS2:XbaI-BamHI-SmaI-KpnI-SacI-EcoRI

# FIG.2

MCS1:HindIII-SphI-PstI
MCS2:SphI-XbaI-BamHI-SmaI-KpnI-SacI-EcoRI

# FIG.3

MCS1:HindIII-PstI
MCS2:XbaI-BamHI-SmaI-KpnI-SacI-EcoRI

# FIG.4

# FIG.5

# FIG.6

MCS2:Xbal-BamHI-Smal-Kpnl-Sacl-EcoRI

# FIG.7

MCS2:XbaI-BamHI-SmaI-KpnI-SacI

EP 0 965 596 A1

FIG.8

FIG.10

25

# FIG.9A

# FIG.9B

# FIG.11

# FIG.12

# FIG.13

# FIG.14

# FIG.15

# FIG.16

# FIG.17

TOTAL TRIGLYCERIDE CONCENTRATION

☐ Control(n=3)
▨ IGF-I 200 $\mu$g/kg(n=3)
▨ IGF-I 50 $\mu$g/kg(n=3)
☐ [Leu27,Leu43]rIGF-II 1,000 $\mu$g/kg(n=3)
■ anti-RBP-3 pAb 20 mg/head(n=3)

*p<0.05

(%)

100

50

0

1 (HOUR)

# FIG.18

TOTAL CHOLESTEROL CONCENTRATION

☐ Control(n=3)
▨ IGF-I 200 $\mu$g/kg(n=3)
▨ IGF-I 50 $\mu$g/kg(n=3)
☐ [Leu27,Leu43]rIGF-II 1,000 $\mu$g/kg(n=3)
■ anti-RBP-3 pAb 20 mg/head(n=3)

(%)

110
100
90
80
70
60
50

6        24        (HOUR)

*p<0.05
**p<0.01

# FIG.19

TOTAL CHOLESTEROL CONCENTRATION

□ Control(n=18)
▨ IGF-I 300 $\mu$ g/kg(n=8)
■ anti-RBP-3 pAb 40 mg/head(n=5)
▦ IGF-II 1,200 $\mu$ g/kg(n=8)
□ IGF-II 600 $\mu$ g/kg(n=8)

***$p < 0.001$

# FIG.20

TOTAL TRIGLYCERIDE CONCENTRATION

□ Control(n=18)
▨ IGF-I 300 $\mu$ g/kg(n=8)
■ anti-RBP-3 pAb 40 mg/head(n=5)
▦ IGF-II 1,200 $\mu$ g/kg(n=8)
□ IGF-II 600 $\mu$ g/kg(n=8)

***$p < 0.001$

# FIG.21

CONCENTRATION OF FREE FATTY ACID

□ Control(n=18)
▨ IGF-I 300 μg/kg(n=8)
■ anti-RBP-3 pAb 40 mg/head(n=5)
▨ IGF-II 1,200 μg/kg(n=8)
□ IGF-II 600 μg/kg(n=8)

# FIG.22

CONCENTRATION OF FREE IGF-I

***P<0.001

□ Control
▨ #90

| INTERNATIONAL SEARCH REPORT | International application No. |
|---|---|
| | PCT/JP97/04881 |

**A. CLASSIFICATION OF SUBJECT MATTER**
    Int.Cl⁶  C07K14/65, A61K45/00, A61K38/30, A61K39/395//A61K31/35,
            A61K31/70, A61K31/725
According to International Patent Classification (IPC) or to both national classification and IPC

**B. FIELDS SEARCHED**

Minimum documentation searched (classification system followed by classification symbols)
    Int.Cl⁶  C07K14/65, A61K45/00, A61K38/30, A61K39/395, A61K31/35,
            A61K31/70, A61K31/725

Documentation searched other than minimum documentation to the extent that such documents are included in the fields searched

Electronic data base consulted during the international search (name of data base and, where practicable, search terms used)
    CAPLUS (STN), REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | YANG, YVONNE W.-H., et al., "Heparin Inhibition of Insulin-Like Growth Factor-Binding Protein-3 Binding to Human Fibroblasts and Rat Glioma Cells: Roll of Heparan Sulfate Proteoglycans", Endocrinology, 1996, Vol. 137, No. 10, p.4363-4371 | 10-19, 25, 28<br>20-24, 26-27 |
| X<br>A | BOOTH, BARBARA A., et al., "Intrinsic Bioactivity of Insulin-Like Growth Factor-Binding Proteins from Vascular Endothelial Cells", Endocrinology, 1990, Vol. 127, No. 6, p.2630-2638 | 10-22, 25-26, 28<br>23-24, 27 |
| X<br>A | JP, 1-104077, A (Hoechst Japan Ltd.),<br>April 21, 1989 (21. 04. 89),<br>Page 17, lower left column<br>& EP, 2528698, A1 & US, 5028698, A | 10-19<br>20-28 |
| X<br>A | JP, 5-52806, B2 (Lion Corp.),<br>August 6, 1993 (06. 08. 93),<br>Claim 1 & EP, 294808, B | 10-19<br>20-28 |

| ☒ Further documents are listed in the continuation of Box C. | ☐ See patent family annex. |
|---|---|

\* Special categories of cited documents:
"A"  document defining the general state of the art which is not considered to be of particular relevance
"E"  earlier document but published on or after the international filing date
"L"  document which may throw doubts on priority claim(s) or which is cited to establish the publication date of another citation or other special reason (as specified)
"O"  document referring to an oral disclosure, use, exhibition or other means
"P"  document published prior to the international filing date but later than the priority date claimed

"T"  later document published after the international filing date or priority date and not in conflict with the application but cited to understand the principle or theory underlying the invention
"X"  document of particular relevance; the claimed invention cannot be considered novel or cannot be considered to involve an inventive step when the document is taken alone
"Y"  document of particular relevance; the claimed invention cannot be considered to involve an inventive step when the document is combined with one or more other such documents, such combination being obvious to a person skilled in the art
"&"  document member of the same patent family

| Date of the actual completion of the international search | Date of mailing of the international search report |
|---|---|
| March 31, 1998 (31. 03. 98) | April 14, 1998 (14. 04. 98) |

| Name and mailing address of the ISA/ | Authorized officer |
|---|---|
| Japanese Patent Office | |
| Facsimile No. | Telephone No. |

Form PCT/ISA/210 (second sheet) (July 1992)

## INTERNATIONAL SEARCH REPORT

| International application No. |
|---|
| PCT/JP97/04881 |

C (Continuation). DOCUMENTS CONSIDERED TO BE RELEVANT

| Category* | Citation of document, with indication, where appropriate, of the relevant passages | Relevant to claim No. |
|---|---|---|
| X<br>A | JP, 53-14605, B2 (Sanyo-Kokusaku Pulp Co., Ltd.),<br>May 18, 1978 (18. 05. 78),<br>Page 1, column 1, line 29 to column 2, line 2<br>(Family: none) | 10-19<br>20-28 |

Form PCT/ISA/210 (continuation of second sheet) (July 1992)

## INTERNATIONAL SEARCH REPORT

| International application No. |
| --- |
| PCT/JP97/04881 |

**Box I   Observations where certain claims were found unsearchable (Continuation of item 1 of first sheet)**

This international search report has not been established in respect of certain claims under Article 17(2)(a) for the following reasons:

1. [x] Claims Nos.: 1-9

   because they relate to subject matter not required to be searched by this Authority, namely:
   Since the term "living bodies" as used in the above claims includes the human body, the inventions as set forth in the above claims are considered to include diagnostic methods practiced on the human body and thus relate to a subject matter which this International Searching

2. [ ] Claims Nos.:

   because they relate to parts of the international application that do not comply with the prescribed requirements to such an extent that no meaningful international search can be carried out, specifically:

3. [ ] Claims Nos.:

   because they are dependent claims and are not drafted in accordance with the second and third sentences of Rule 6.4(a).

**Box II   Observations where unity of invention is lacking (Continuation of item 2 of first sheet)**

This International Searching Authority found multiple inventions in this international application, as follows:
(A) The group of inventions of Claims 1 to 3 pertains to methods for elevating the concentration of a free insulin-like growth factor from a two-membered complex composed of an insulin-like growth factor and an insulin-like growth factor-binding protein in living bodies.
(B) The group of inventions of Claims 4 to 6 pertains to methods for elevating the concentration of the two-membered complex composed of an insulin-like growth factor and an insulin-like growth factor-binding protein from a three-membered complex composed of an insulin-like growth factor, an insulin-like growth factor-binding protein and subunits unstable to acids in living bodies.

1. [x] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**   [ ]   The additional search fees were accompanied by the applicant's protest.

[ ]   No protest accompanied the payment of additional search fees.

Form PCT/ISA/210 (continuation of first sheet (1)) (July 1992)

INTERNATIONAL SEARCH REPORT

| International application No. |
| --- |
| PCT/JP97/04881 |

Continuation of Box No. I of continuation of first sheet (1)

Authority is not required, under the provisions of Article 17(2)(a)(i) of the PCT and Rule 39.1(iv) of the Regulations under the PCT, to search.

Continuation of Box No. II of continuation of first sheet (1)

(C) The group of inventions of Claims 7 to 9 pertains to methods for elevating the concentration of a free insulin-like growth factor from the three-membered complex composed of an insulin-like growth factor, an insulin-like growth factor-binding protein and subunits unstable to acids in living bodies.
(D) The group of inventions of Claims 10 to 12 pertains to a substance capable of forming a free insulin-like growth factor from the two-membered complex composed of an insulin-like growth factor and an insulin-like growth factor-binding protein in living bodies.
(E) The group of inventions of Claims 13 to 15 pertain to a substance capable of forming the two-membered complex composed of an insulin-like growth factor and an insulin-like growth factor-binding protein from the three-membered complex composed of an insulin-like growth factor, an insulin-like growth factor-binding protein and subunits unstable to acids in living bodies.
(F) The group of inventions of Claims 16 to 18 pertains to a substance capable of forming a free insulin-like growth factor from the three-membered complex composed of an insulin-like growth factor, an insulin-like growth factor-binding protein and subunits unstable to acids in living bodies.
(G) The group of inventions of Claims 19 to 24 pertains to a substance binding substantially neither to an insulin-like growth factor receptor nor an insulin receptor but to an insulin-like growth factor-binding protein.
(H) The invention of Claim 25 pertains to drugs containing the substance as claimed in any of Claims 10 to 19.
(I) The group of inventions of Claims 26 and 27 pertains to drugs containing the substance as claimed in any of Claims 20 to 24.
(J) The invention of Claim 28 pertains to a method for screening the substance as claimed in any of Claims 10 to 19.

A combination of two groups (D) and (A) of inventions, a combination of two groups (E) and (B) of inventions, and a combination of two groups (F) and (C) of inventions can be regarded each as a combination of a product with the use thereof and thus considered as forming a group of inventions.
The groups (D) and (E) of inventions aim at achieving the same object and have a principal part in common of forming a free insulin-like growth factor from the two-membered complex composed of an insulin-like growth factor and an insulin-like growth factor-binding protein. From this viewpoint, they are also considered as forming a group of inventions.
Such being the case, these groups (A), (C), (D) and (F) of inventions are considered as relating to a group of inventions so linked as to form a single general inventive concept.
However, the groups (A), (C), (D) and (F) of inventions and the groups (B) and (E) of inventions are not considered as aiming at achieving the same object and as having a special technical matter in common. Thus, at least the groups (A), (C), (D) and (F) of inventions and the groups (B) and (E) of inventions are not considered as relating to a group of inventions so linked as to form a single general inventive concept.
Similarly, the group of inventions of Claims 20 to 24 and the group (I) of inventions can be regarded as a combination of a product with the use thereof and, therefore, the groups (G) and (I) of inventions can be considered as forming a group of inventions.

Form PCT/ISA/210 (extra sheet) (July 1992)

## INTERNATIONAL SEARCH REPORT

International application No.

PCT/JP97/04881

Further, the groups (A), (C), (D) and (F) of inventions and the groups (G) and (I) of inventions are not considered as aiming at achieving the same object and as having a special technical matter in common. Thus, at least the groups (A), (C), (D) and (F) of inventions and the groups (G) and (I) of inventions are not considered as relating to a group of inventions so linked as to form a single general inventive concept.

The same applies to the relation between the groups (B) and (E) of inventions and the groups (G) and (I) of inventions.

The above inventions (H) and (J) are each described by citing the inventions of Claims 10 to 19 failing to form a group of inventions. Therefore these inventions are not considered as relating to a group of inventions so linked as to form a single general inventive concept.

Such being the case, the inventions as will be specified below do not have any special technical feature in common to each other and are not considered as relating to a group of inventions so linked as to form a single general inventive concept:

I: the groups (A), (C), (D) and (F) of inventions (i.e., the groups of inventions of Claims 1 to 3, 7 to 9, 10 to 12, and 16 to 18);

II: the groups (B) and (E) of inventions (i.e., the groups of inventions of Claims 4 to 6, and 13 to 15);

III: the groups (G) and (I) of inventions (i.e., the groups of inventions of Claims 19 to 24, 26 and 27);

IV: the above invention (H) (i.e., the invention of Claim 25); and

V: the above invention (J) (i.e., the invention of Claim 28).

Form PCT/ISA/210 (continuation of extra sheet) (July 1992)